(19) Europäisches Patentamt
European Patent Office
Office européen des brevets

(11) **EP 2 565 904 B1**

(12) **FASCICULE DE BREVET EUROPEEN**

(45) Date de publication et mention
de la délivrance du brevet:
**21.10.2015 Bulletin 2015/43**

(51) Int Cl.:
*H01J 49/00* *(2006.01)*   *G01N 30/72* *(2006.01)*
*G01N 29/02* *(2006.01)*   *G06F 19/24* *(2011.01)*
*B82Y 15/00* *(2011.01)*

(21) Numéro de dépôt: **12183031.9**

(22) Date de dépôt: **04.09.2012**

(54) **Procédé et dispositif d'estimation d'un paramètre de masse moléculaire dans un échantillon**

Verfahren und Vorrichtung zum Schätzen eines Molekülmassenparameters in einer Probe

Method and apparatus for estimating a molecular mass parameter in a sample

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **05.09.2011 FR 1157857**

(43) Date de publication de la demande:
**06.03.2013 Bulletin 2013/10**

(73) Titulaire: **Commissariat à l'Énergie Atomique et aux Énergies Alternatives
75015 Paris (FR)**

(72) Inventeurs:
• **Perenon, Rémi**
**38500 Voiron (FR)**
• **Grangeat, Pierre**
**38330 Saint Ismier (FR)**
• **Mohammad-Djafari, Ali**
**91400 Orsay (FR)**

(74) Mandataire: **Bonnet, Michel**
**Cabinet Bonnet**
**93, rue Réaumur - Boîte 10**
**75002 Paris (FR)**

(56) Documents cités:
**EP-A2- 1 047 108**     **FR-A1- 2 920 235**
**US-A1- 2007 023 621**     **US-A1- 2009 261 241**

• GRUBER D ET AL: "Counting of obscure events: A Bayesian approach", CHEMICAL PHYSICS LETTERS, vol. 474, no. 4-6, 4 juin 2009 (2009-06-04), pages 371-374, XP026140398, ISSN: 0009-2614, DOI: 10.1016/J.CPLETT.2009.04.060 [extrait le 2009-05-03]
• SCHWARZ-SELLINGER T ET AL: "Analysis of multicomponent mass spectra applying Bayesian probability theory", JOURNAL OF MASS SPECTROMETRY, vol. 36, 1 août 2001 (2001-08-01), pages 866-874, XP002476267, ISSN: 1076-5174, DOI: 10.1002/JMS.187 [extrait le 2001-08-07]

**Description**

La présente invention concerne un procédé et un dispositif d'estimation d'un paramètre de masse moléculaire dans un échantillon.

Une application particulièrement prometteuse d'un tel procédé est par exemple l'analyse d'échantillons biologiques tels que des prélèvements sanguins ou de plasma pour en extraire des paramètres biologiques tels qu'une estimation de spectre de masse permettant de remonter à des concentrations moléculaires en protéines. La connaissance de ce spectre de masse et/ou ces concentrations permet de détecter des anomalies ou maladies. Notamment, il est connu que certaines maladies telles que des cancers, même à un stade peu avancé, peuvent avoir un impact éventuellement décelable sur les concentrations moléculaires de certaines protéines. L'étude de ces protéines, appelée protéomique, devient ainsi un élément incontournable dans de nombreux domaines de la médecine. En effet, les protéines sont des cas particulièrement intéressants de biomarqueurs qui décrivent à la fois l'expression des gènes, l'influence de l'environnement et sont de plus accessibles dans un bon nombre de fluides organiques (sang, sérum, urine, fluide biologique, lysat d'un échantillon biologique, ...). Mais plus généralement, l'analyse d'échantillons pour l'extraction de paramètres de masse moléculaire pertinents permettant par exemple une aide au diagnostic d'un état (santé, pollution, ...) pouvant être associé à ces échantillons est un domaine d'application prometteur d'un procédé selon l'invention.

Parmi les applications concrètes envisageables, on peut noter les suivantes : l'analyse biologique d'échantillons par détection de protéines et de leurs masses ; la caractérisation de bactéries par spectrométrie de masse ; la caractérisation de l'état de pollution d'un échantillon chimique (par exemple, le dosage d'un gaz dans un environnement ou le dosage d'un métal lourd dans un échantillon liquide). Les paramètres pertinents extraits peuvent comporter des spectres de masses de composants tels que des molécules (peptides, protéines, enzymes, anticorps, ...) ou des assemblages moléculaires. Par assemblage moléculaire on entend par exemple une nanoparticule ou une espèce biologique (bactérie, microorganisme, cellule, ...).

Dans le cas d'une analyse biologique par détection de protéines, toute la difficulté est de parvenir à une estimation la plus précise possible dans un environnement bruité où les protéines d'intérêt sont parfois présentes dans l'échantillon en très petite quantité.

En général, l'échantillon passe par une chaîne de traitement comportant au moins un spectromètre de masse. Cette chaîne de traitement est conçue pour la fourniture d'un signal représentatif de masses des composants dans l'échantillon en fonction d'un rapport masse/charge dans le spectromètre de masse.

Eventuellement, la chaîne de traitement peut comporter, en amont du spectromètre de masse imposant que l'échantillon soit en phase gazeuse nébulisée, un vaporisateur et un électrospray (ou équivalent, pour réaliser une ionisation et/ou désorption des composants de l'échantillon) apte à procéder au changement de phase nécessaire lorsque l'échantillon à analyser est par exemple disponible en phase liquide ou solide.

Enfin, lorsque le spectromètre de masse utilisé est à capteur électromécanique de type MEMS (de l'anglais « Micro Electro Mechanical System ») ou NEMS (de l'anglais « Nano Electro Mechanical System »), la chaîne de traitement peut éventuellement comporter une électronique de focalisation, par exemple un hexapôle, guidant les composants ionisés à travers une chambre à très basse pression. Les capteurs de type MEMS sont utilisés pour des composés de masses moléculaires importantes, par exemple des assemblages moléculaires, tandis que les capteurs de type NEMS sont utilisés pour détecter des composés de masses moléculaires plus faibles, par exemple des molécules uniques.

L'intérêt majeur d'utiliser un spectromètre de masse à capteur électromécanique de type MEMS ou NEMS dans la chaîne de traitement est de permettre une quantification et une estimation du paramètre de masse (par exemple la masse ou le spectre de masse) au niveau du composant unique. La tâche de spectrométrie de masse est alors effectuée en mode comptage, ce qui donne lieu à une grande sensibilité et une réduction du bruit de mesure.

Un procédé connu d'estimation d'un paramètre de masse moléculaire dans un échantillon utilisant un tel spectromètre de masse à capteur MEMS ou NEMS est décrit dans l'article de Naik et al, intitulé « Towards single-molecule nanomechanical mass spectrometry », publié dans Nature Nanotechnology, vol. 4, pages 445-450, Juillet 2009. Il comporte les étapes suivantes :

-    faire passer l'échantillon par une chaîne de traitement comportant un spectromètre de masse à capteur électromécanique de type MEMS ou NEMS,
-    obtenir ainsi un signal représentatif du paramètre de masse moléculaire en fonction d'au moins une variable de la chaîne de traitement, et en particulier du capteur électromécanique, et
-    estimer le paramètre de masse moléculaire à l'aide d'un dispositif de traitement de signal.

Dans ce document, le principe est plus précisément le suivant :

-    des molécules sont vaporisées, puis ionisées par électrospray,

- ces molécules ionisées sont guidées à travers un hexapôle,
- les molécules sont ensuite bombardées sur un capteur NEMS du spectromètre de masse,
- un système de lecture électronique vient lire certaines caractéristiques du capteur,
- un traitement de signal permet d'estimer le nombre de molécules en amont de la chaîne de traitement.

Concrètement, les molécules sont adsorbées sur le capteur NEMS. Ce capteur, se comportant comme un système masse-ressort, voit sa fréquence de résonance chuter en fonction de la masse adsorbée. Le système de lecture électronique, qui est en l'occurrence une boucle à verrouillage de phase, vient lire cette fréquence de résonance.

Le traitement de signal proposé dans ce document est alors effectué selon les étapes suivantes :

- estimation préalable de l'écart-type du bruit en ne bombardant pas le capteur NEMS,
- détection d'instants d'adsorption de molécules bombardées sur le capteur NEMS en dérivant numériquement le signal observé et en ne gardant que les valeurs de la dérivée supérieures à deux fois l'écart-type estimé préalablement,
- estimation de chutes de fréquence aux instants précédemment détectés via une estimation basée sur la méthode des moindres carrés,
- transformation de l'estimation des chutes de fréquence en estimation de masses moléculaires.

Cette méthode d'estimation reste très expérimentale et approximative. De plus, elle est réalisée globalement sur l'ensemble des chutes en fréquence. Il n'y a pas d'estimation molécule par molécule.

Il peut ainsi être souhaité de prévoir un procédé d'estimation d'un paramètre de masse moléculaire qui permette de s'affranchir d'au moins une partie des problèmes et contraintes précités et d'améliorer les procédés existants.

Il est donc proposé un procédé d'estimation d'un paramètre de masse moléculaire dans un échantillon selon la revendication 1.

Le document US 2007/023621 décrit un système de spectrométrie de masse présentant un capteur électromécanique de type MEMS ou NEMS En effet, ce document divulgue toutes les caractéristiques du préambule de la revendication 1.

GRUBER D et al: "Counting of obscure events: A Bayesian approach" CHEMICAL PHYSICS LETTERS, vol. 474, no. 4-6, le 4 juin 2009, pages 371-374, XP026140398, ISSN 0009-2614 divulgue une méthode d'estimation par inférence bayésienne tirant parti de connaissances à priori sur le système physique observé pour compter des événements. Ces événements peuvent en particulier être des molécules identifiées dans une chambre de détection par un photodétecteur.

Ainsi, la modélisation analytique proposée rend le signal observé statistiquement dépendant du paramètre de masse et d'un ensemble de paramètres techniques de la chaîne de traitement dont certains directement liés à la présence d'un spectromètre de masse à capteur MEMS ou NEMS. La connaissance, a priori incomplète, de certains de ces paramètres peut être modélisée par des lois de probabilités, tandis que d'autres sont obtenus de façon déterministe, par apprentissage ou éventuellement par calibrage de la chaîne de traitement. Une inversion par inférence bayésienne du modèle statistique obtenu permet alors d'obtenir une estimation simple et fiable du paramètre de masse moléculaire considéré, à partir du signal observé en sortie de la chaîne de traitement.

De façon avantageuse, le paramètre de masse moléculaire est défini sur la base d'un paramètre de répartition temporelle de détections successives, par le capteur électromécanique de type MEMS ou NEMS, de l'adsorption dudit composant.

On peut alors déduire la masse dudit composant, ou de chaque composant constituant l'échantillon, ainsi que le nombre de détections dudit composant.

De façon optionnelle, la modélisation analytique comprend la définition d'un paramètre représentant les instants, dits instants de détection, auxquels le capteur électromécanique détecte l'adsorption dudit composant. Dans ce cas, le paramètre de masse moléculaire est avantageusement défini à partir de ce paramètre représentant les instants de détection.

De façon optionnelle également, le paramètre représentant les instants de détection prend la forme d'un vecteur ou d'une liste des paramètres de chaque détection de composant.

De façon optionnelle également, un procédé selon l'invention peut en outre comporter une étape de détection des lieux d'adsorption des composants sur le capteur électromécanique de type MEMS ou NEMS, auquel cas la modélisation analytique comporte en outre un paramètre de répartition temporelle de ces lieux d'adsorption et une fonction déterministe donnant une valeur de chute de fréquence pour chaque couple de valeurs d'une masse de composant adsorbée et d'un lieu d'adsorption correspondant.

De façon optionnelle également, au moins deux des paramètres de masse moléculaire et de la chaîne de traitement en fonction desquels la modélisation analytique directe dudit signal est définie ont une relation de dépendance probabiliste entre eux, et le traitement de signal par inférence bayésienne est en outre réalisé sur la base d'une modélisation par loi de probabilité a priori conditionnelle de cette dépendance.

Ainsi, la modélisation peut être affinée, et donc s'approcher de la réalité, en intégrant une hiérarchie entre signaux ou entre des signaux et des paramètres à l'aide de dépendances probabilistes traduites par des lois de probabilités conditionnelles, sachant que ces dépendances probabilistes peuvent être modélisées a priori, par exemple soit par un apprentissage spécifique, soit par un modèle réaliste imposé par l'expérience. Il en résulte finalement une meilleure estimation du paramètre de masse moléculaire concerné.

De façon optionnelle également, l'étape d'estimation du paramètre de masse moléculaire comporte, par approximation de la loi de probabilité a posteriori jointe d'un paramètre lié au paramètre de masse moléculaire et des paramètres techniques de la chaîne de traitement conditionnellement au signal obtenu à l'aide d'un algorithme d'échantillonnage stochastique :

- une boucle d'échantillonnage de ces paramètres, fournissant des valeurs échantillonnées de ces paramètres, et
- une estimation du paramètre de masse moléculaire calculée à partir des valeurs échantillonnées fournies.

Ainsi, sur la base d'une connaissance de modèles de lois de probabilités a priori, conditionnelles ou non, d'au moins une partie de ces paramètres, il devient possible de traiter simplement le signal fourni par la chaîne de traitement pour en extraire des estimations de ces paramètres.

De façon optionnelle également :

- le paramètre représentant les instants de détection prend la forme d'un vecteur à composantes binaires, l'une des valeurs binaires, A, indiquant la détection d'une adsorption,
- à chaque itération de la boucle d'échantillonnage, un échantillon de ce paramètre est calculé dans un voisinage de l'échantillon calculé à l'itération précédente,
- le voisinage d'un échantillon courant de ce paramètre est défini de la façon suivante : tout échantillon comportant une composante à A en plus ou en moins, une composante à A décalée, ou une composante A regroupant deux composantes à A successives de l'échantillon courant.

De façon optionnelle également :

- à chaque itération de la boucle d'échantillonnage, une probabilité jointe d'au moins tous les paramètres échantillonnés est estimée, et
- l'estimation du paramètre de masse moléculaire se fait, conjointement avec celle des paramètres échantillonnés, sur la base des valeurs successives de ladite probabilité jointe.

De façon optionnelle également, le paramètre de masse moléculaire est un spectre de masse relatif à au moins un composant dont la masse fait partie des paramètres traités par la boucle d'échantillonnage et est l'un des éléments de l'ensemble constitué d'une unique masse moléculaire, d'une pluralité discrète de masses moléculaires et d'une distribution continue de masses.

De façon optionnelle également, le paramètre de masse moléculaire est relatif à des protéines et l'échantillon comporte l'un des éléments de l'ensemble constitué de sang, plasma, urine, fluide biologique et lysat d'un échantillon biologique.

L'invention a également pour objet un dispositif d'estimation d'un paramètre de masse moléculaire dans un échantillon, selon la revendication 11.

L'invention sera mieux comprise à l'aide de la description qui va suivre, donnée uniquement à titre d'exemple et faite en se référant aux dessins annexés dans lesquels :

- la figure 1 représente schématiquement la structure générale d'un dispositif d'estimation d'un paramètre de masse moléculaire selon un mode de réalisation de l'invention,
- la figure 2 illustre une première modélisation analytique d'une chaîne de traitement du dispositif de la figure 1, selon un premier mode de réalisation de l'invention,
- la figure 3 illustre les étapes successives d'un procédé d'estimation d'un paramètre de masse moléculaire mis en oeuvre par un dispositif de traitement de signal du dispositif de la figure 1,
- la figure 4 illustre une deuxième modélisation analytique d'une chaîne de traitement du dispositif de la figure 1, selon un deuxième mode de réalisation de l'invention,
- la figure 5 illustre une troisième modélisation analytique d'une chaîne de traitement du dispositif de la figure 1, selon un troisième mode de réalisation de l'invention, et
- la figure 6 illustre le détail d'une étape du procédé d'estimation de la figure 3, selon le troisième mode de réalisation de l'invention.

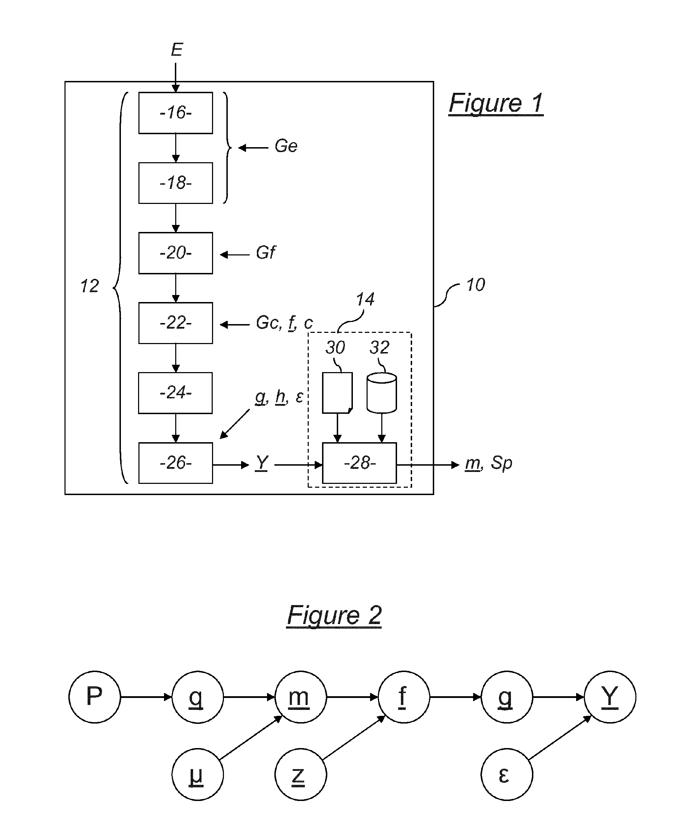

Le dispositif 10 d'estimation d'un paramètre de masse moléculaire dans un échantillon E, représenté schématiquement sur la figure 1, comporte une chaîne 12 de traitement de l'échantillon E conçue pour la fourniture d'un signal Y représentatif de ce paramètre de masse moléculaire en fonction d'au moins une variable de la chaîne de traitement 12. Il comporte en outre un dispositif 14 de traitement de signal conçu pour appliquer, en combinaison avec la chaîne de traitement 12, un procédé d'estimation de ce paramètre de masse moléculaire en fonction des paramètres techniques de la chaîne de traitement 12.

Dans l'exemple qui va être détaillé par la suite mais qui ne doit pas être considéré comme limitatif, le paramètre estimé est un spectre de masse relatif à des composants biologiques de l'échantillon E considéré alors comme un échantillon biologique, et la chaîne de traitement 12 est une chaîne de traitement biologique. Plus précisément, les composants sont des protéines d'intérêt, par exemple sélectionnées en fonction de leur pertinence pour caractériser une anomalie, affection ou maladie, et l'échantillon E est un échantillon de sang, de plasma, d'urine, de tout autre fluide biologique ou d'un lysat d'échantillon biologique.

Dans la chaîne de traitement biologique 12, l'échantillon E passe tout d'abord par une source d'ions, c'est-à-dire un vaporisateur 16, puis un ionisateur 18 (électrospray ou équivalent), de manière à se présenter en phase gazeuse nébulisée. Cette source d'ions 16, 18 présente un gain Ge qui peut, selon un modèle simplifié de la chaîne de traitement 12, être considéré comme constant et indépendant de la masse des molécules présentes dans l'échantillon E. Ce gain Ge peut être qualifié de paramètre certain dans le sens où il peut être considéré comme constant d'un traitement biologique à l'autre.

L'échantillon E traverse ensuite un hexapôle 20 de focalisation, guidant les molécules ionisées à travers une chambre à très basse pression. Cette focalisation présente également un gain Gf qui peut, selon un modèle simplifié de la chaîne de traitement 12, être considéré comme constant et indépendant de la masse des molécules présentes dans l'échantillon E. Ce gain Gf peut être également qualifié de paramètre certain.

Puis les molécules ionisées et focalisées de l'échantillon E rencontrent le capteur électromécanique de type MEMS ou NEMS d'un spectromètre de masse 22 par exemple similaire à celui qui est décrit dans l'article précité de Naik et al. Elles sont adsorbées par ce capteur selon un gain Gc qui peut, selon un modèle simplifié de la chaîne de traitement 12, être considéré comme constant et indépendant de la masse des molécules présentes dans l'échantillon E. Ce gain Gc peut être également qualifié de paramètre certain.

Pour chaque molécule adsorbée par le capteur MEMS ou NEMS du spectromètre de masse 22, une chute de fréquence propre de résonance du capteur peut être observée, cette chute de fréquence étant dépendante de l'endroit où la molécule a été adsorbée sur le capteur et de sa masse. En effet, la fréquence propre de résonance du capteur dépend de sa masse effective, laquelle dépend non pas uniquement de la masse totale mais de la distribution de masse le long du capteur. Pour mieux paramétrer la fonction qui définit cette dépendance de la fréquence propre de résonance du capteur MEMS ou NEMS au lieu d'absorption et à la masse adsorbée, la chaîne de traitement 12 peut éventuellement comporter un dispositif 24 de détection de la position de la dernière molécule adsorbée sur le capteur MEMS ou NEMS. On considère qu'un tel dispositif 24 est connu, il ne sera donc pas détaillé. On pourra notamment se référer à l'article de Dohn et al, intitulé « Mass and position determination of attached particles on cantilever based mass sensors », publié en ligne dans Review of scientific intruments 78, 103303, 31 octobre 2007.

Selon une variante, le capteur MEMS ou NEMS est fonctionnalisé localement, ce qui permet de prédéterminer la position de toute adsorption. La fonctionnalisation peut être réalisée sur une zone suffisamment étroite, de telle sorte que la variabilité du lieu d'absorption n'influe pas sur la mesure.

Enfin, la chaîne de traitement 12 comporte un système 26 de lecture électronique des chutes de fréquence du capteur MEMS ou NEMS du spectromètre de masse 22 pour la fourniture d'un signal observé Y qui est alors représentatif des masses moléculaires des protéines dans l'échantillon E. Ce signal Y correspond à l'enregistrement de la fréquence de résonance instantanée du capteur MEMS ou NEMS en fonction du temps.

Pour déterminer les paramètres certains et autres constantes de la chaîne de traitement, celle-ci peut être calibrée.

Un premier type de calibrage, dit calibrage externe, peut être réalisé à l'aide d'au moins un échantillon $E_{CALIB}$ de composants de calibrage externe dont le profil moléculaire est connu, par exemple un échantillon de protéines standard ou de protéines prédéterminées contenues dans des cocktails de protéines, la masse moléculaire de ces protéines de calibrage étant connue et leur concentration (ou leur nombre) dans l'échantillon $E_{CALIB}$ étant également connue.

Un second type de calibrage, dit calibrage interne, peut être réalisé en incorporant dans l'échantillon E à analyser au moins un autre échantillon E* de composants connus (dans l'exemple choisi, ce sont des protéines) de marquage (équivalents aux composants d'intérêt mais de masse différente connue, leur concentration ou leur nombre dans l'échantillon E* étant également connus).

La chaîne de traitement 12 peut être calibrée par calibrage interne ou externe, l'un n'excluant pas l'autre. En particulier, le produit des trois gains précités Ge Gf et Gc peut être déterminé par calibrage interne.

Une modélisation analytique directe du signal Y va maintenant être proposée, dans laquelle interviennent au moins un paramètre lié à la masse moléculaire des composants de l'échantillon E et des paramètres techniques de la chaîne de traitement. Cette modélisation est construite autour des spécificités du capteur MEMS ou NEMS du spectromètre de masse 22. Ainsi, seules les molécules effectivement adsorbées sur ce capteur sont prises en compte dans le modèle. La composition réelle de l'échantillon E fourni en entrée de la chaîne de traitement 12 peut cependant être aisément retrouvée à partir du nombre et de la masse des molécules adsorbées ainsi que des paramètres de gains connus.

Afin de pouvoir appliquer au signal Y des méthodes de traitements statistiques adéquates, les adsorptions de molécules sur le capteur MEMS ou NEMS du spectromètre de masse 22 sont comptées dans des fenêtres d'observation de T échantillons réguliers et les paramètres sont exprimés par des vecteurs discrétisés à T composantes. Ainsi par exemple, le signal observé Y devient un vecteur $\underline{Y}$ discrétisé à T composantes.

Dans ces mêmes fenêtres d'observation, on note m la répartition temporelle des masses adsorbées. En temps continu et selon le modèle choisi, cette répartition temporelle est un peigne pondéré de Dirac s'exprimant sous la forme

$$m(t) = \sum_{i=1}^{I} M_i \cdot \delta(t - t_i),$$ où I est le nombre de molécules (ou d'assemblages moléculaires) détectées comme adsorbées dans une fenêtre d'observation, chaque détection d'adsorption de molécule (ou d'assemblage moléculaire) étant qualifiée d'évènement, $M_i$ les masses respectives des I molécules adsorbées, $\delta$ la distribution de Dirac et $t_i$ les instants respectifs d'adsorption des molécules (ou assemblages moléculaires). En temps discret, cette répartition devient un vecteur $\underline{m}$ à T composantes, dont I composantes non nulles. Cette répartition temporelle peut également s'exprimer en variante sous la forme d'une liste m comprenant, pour chaque évènement, la masse détectée ($M_i$) et l'instant de détection ($t_i$). Autrement dit, lorsque m représente une liste, $m = \{(M_i, t_i)\}$.

On note en outre z la répartition temporelle des lieux d'adsorption. En temps continu et selon le modèle choisi, cette répartition temporelle est un signal continu par morceaux de Dirac s'exprimant sous la forme :

$$z(t) = 0 \text{ si } t < t_1,$$

$$z(t) = z_i \text{ si } t \in [t_i ; t_{i+1}[,$$

$$z(t) = z_I \text{ si } t > t_I,$$

où les $z_i, 1 \leq i \leq I$ sont les lieux respectifs d'adsorption. En temps discret, cette répartition devient un vecteur $\underline{z}$ à T composantes. Ce paramètre $\underline{z}$ peut être qualifié de paramètre incertain dans le sens où il est susceptible de varier de façon aléatoire d'un traitement biologique à l'autre. Mais étant donné qu'à chaque instant on peut connaître la position de la dernière molécule adsorbée grâce au dispositif 24 de détection de position, il n'y a pas besoin de le modéliser par une loi de probabilité a priori.

La chute de fréquence induite dans le capteur MEMS ou NEMS à chaque adsorption est dépendante des caractéristiques électromécaniques de ce capteur selon une fonction déterministe notée c de chaque couple ($M_i, z_i$). Un exemple de telle fonction peut être déduit de l'article précité de Dohn et al. Les valeurs que prend cette fonction à chaque instant de détection $t_i$ peuvent être notées $c(M_i, z_i)$. Idéalement, le capteur MEMS ou NEMS du spectromètre de masse 22 est choisi de manière à être suffisamment sensible pour détecter une chute de tension notable à chaque adsorption et suffisamment rapide pour discriminer temporellement les chutes de fréquences successives dues aux adsorptions successives. Ainsi, chaque chute de fréquence observable peut être considérée comme immédiate et parfaite.

Par la suite, on notera :

$$c(\underline{m}, \underline{z}) = \sum_{i=1}^{I} c(M_i, z_i) \cdot \delta(t - t_i),$$

L'évolution de la fréquence propre de résonance du capteur MEMS ou NEMS du spectromètre de masse 22 dans une fenêtre d'observation peut alors s'exprimer sous la forme suivante :

$$f(t) = f_0 - \int_0^t \left( \sum_{i=1}^{I} c(M_i, z_i) \cdot \delta(u - t_i) \right) du .$$

En temps discret, cette fréquence de résonance devient un vecteur $\underline{f}$ dont les composantes s'écrivent sous la forme :

$$\underline{f}(t) = f_0 - \sum_{u=1}^{t} \sum_{i=1}^{I} c(M_i, z_i) \cdot \delta(u - t_i) .$$

Par conséquent, le signal théorique $\underline{g}$ fourni en sortie du système 26 de lecture électronique peut s'écrire comme le produit de convolution de la fréquence de résonance $\underline{f}$ précitée par le filtre $\underline{h}$ que représente la boucle de lecture de ce système 26. En temps discret, la relation s'écrit $\underline{g} = \underline{f} * \underline{h}$.

Il suffit de connaître $f_0$ (ou l'équivalent $g_0$) ainsi que les fonctions c et $\underline{h}$ pour que les sorties $\underline{f}$ et $\underline{g}$ soient complètement déterminées.

Enfin, pour tenir compte des imperfections de modèle ainsi que des perturbations, on considère que le signal observé $\underline{Y}$ est lié à $\underline{g}$ par l'ajout d'un bruit de paramètre $\varepsilon$, ce paramètre étant par exemple la variance du bruit. Ce paramètre $\varepsilon$ peut être qualifié de paramètre incertain dans le sens où il est susceptible de varier de façon aléatoire d'un traitement biologique à l'autre. Il peut donc avantageusement être modélisé selon une loi de probabilité a priori prédéterminée, par exemple une loi inverse-gamma $IGAM(\varepsilon | k_\varepsilon, T_\varepsilon)$ de forme $k_\varepsilon$ et d'intensité $T_\varepsilon$, ces deux derniers paramètres représentant la connaissance a priori sur le bruit notée $\theta_\varepsilon$.

Le signal observé $\underline{Y}$ est fourni en entrée du dispositif de traitement 14. Plus précisément, le dispositif de traitement 14 comporte un processeur 28 relié à des moyens de stockage comportant notamment au moins une séquence programmée d'instructions 30 et une base de données de modélisation 32.

La base de données 32 comporte les paramètres d'une modélisation analytique directe du signal $\underline{Y}$ en fonction :

- du paramètre de masse moléculaire $\underline{m}$ qui permet de remonter à une connaissance du spectre de masse Sp des composants de l'échantillon E,
- du paramètre $\underline{z}$ de répartition temporelle des lieux d'adsorption sur le capteur MEMS ou NEMS,
- des paramètres techniques précités Ge, Gf, Gc, $f_0$, $g_0$, $\varepsilon$ de la chaîne de traitement biologique 12,
- des fonctions c, $\underline{f}$, $\underline{h}$ et $\underline{g}$, caractéristiques elles aussi de la chaîne de traitement biologique 12, et
- d'autres paramètres théoriques permettant d'affiner l'explicitation du paramètre $\underline{m}$ dans différents modes de réalisation qui seront détaillés ci-dessous.

Sur fourniture du signal effectivement observé $\underline{Y}$, la séquence programmée d'instructions 30 est conçue pour résoudre l'inversion de ce modèle analytique dans un cadre bayésien par une estimation a posteriori basée sur des modèles de probabilité, par exemple des modèles a priori, d'au moins une partie des paramètres précités.

La séquence d'instructions 30 et la base de données 32 sont fonctionnellement présentées comme distinctes sur la figure 1, mais en pratique elles peuvent êtres réparties différemment en fichiers de données, codes sources ou librairies informatiques sans que cela ne change quoi que ce soit aux fonctions remplies.

Dans un premier mode de réalisation, dans lequel on suppose que les molécules de l'échantillon présentent une distribution continue de masses, le paramètre $\underline{m}$ peut être affiné en introduisant un paramètre théorique $\underline{q}$ à valeurs binaires représentant chaque adsorption sur le capteur MEMS ou NEMS. Ce paramètre est un vecteur dont les composantes prennent la forme :

$$\underline{q}(t) = \sum_{i=1}^{I} \delta(t - t_i) .$$

On rappelle que la variable $t_i$ désigne les instants respectifs d'adsorptions, une adsorption correspondant à la détection d'une molécule ou d'un assemblage moléculaire sur le capteur MEMS ou NEMS. Ainsi, la variable $\underline{q}$ prend en compte le caractère discret de la détection des molécules ou assemblages moléculaires par un capteur, ce qui constitue une particularité des capteurs MEMS ou NEMS.

De manière équivalente à ce qui a été précisé pour $\underline{m}$, la connaissance de ces instants d'adsorption $t_i$, et de la masse détectée à chacun de ces instants permet de constituer une liste d'évènements, chaque évènement correspondant à la détection d'une molécule ou d'un assemblage moléculaire. Typiquement, on attribue, à chaque évènement d'indice i, un couple $(M_i, t_i)$, représentant la masse $M_i$ détectée à l'instant $t_i$. La liste est constituée par l'ensemble de ces couples, chaque couple étant associé à une détection. Le formalisme d'une telle liste prend en compte le caractère discret de la détection des molécules ou assemblages moléculaires par un capteur, caractère spécifique des capteurs MEMS ou NEMS, comme précédemment indiqué.

Le paramètre $\underline{m}$ ne prend de valeur non nulle que lorsque $\underline{q}$ est non nul. Par conséquent, $\underline{m}$ peut s'exprimer sous la forme $\underline{m} = \underline{\mu} \cdot \underline{q}$ où $\underline{\mu}$ représente l'estimation de la masse continue des molécules à chaque instant. Les valeurs de $\underline{\mu}$ n'ont de sens que là où les valeurs de $\underline{q}$ ne sont pas nulles.

Dans le modèle choisi, le paramètre $\underline{q}$ est incertain et lui-même soumis à un autre paramètre incertain P théorique indiquant la probabilité que les composantes de $\underline{q}$ prennent la valeur 1 dans une fenêtre d'observation. En d'autres termes, le paramètre P est un scalaire qui modélise la densité temporelle des adsorptions. Il peut avantageusement être modélisé selon une loi de probabilité a priori prédéterminée, par exemple une loi bêta $BET(P|a_P,b_P)$ de paramètres de forme $a_P$ et $b_P$, ces deux derniers paramètres représentant la connaissance a priori sur P notée $\theta_P$. Quant au vecteur $\underline{q}$, il peut être modélisé par une loi de Bernoulli, la probabilité que $\underline{q}$ vaille 1 à chaque instant étant donnée par le paramètre P.

Dans le modèle choisi également, le paramètre vectoriel $\underline{\mu}$ est incertain et ses T composantes indépendantes $\mu_k$ sont modélisées selon une loi de probabilité a priori prédéterminée, par exemple une loi gamma $GAM(\mu_k|k_m,T_m)$ de forme $k_m$ et d'intensité $T_m$, ces deux derniers paramètres représentant la connaissance a priori sur le paramètre de masse moléculaire notée $\theta_m$.

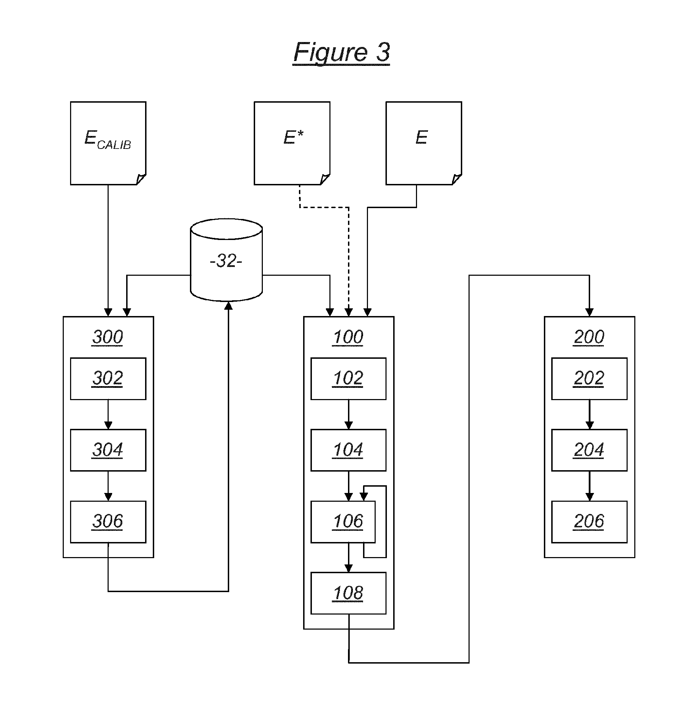

En résumé, certains des paramètres ou fonctions précités sont incertains ou dépendants de paramètres incertains et sont éventuellement, pour une partie d'entre eux, modélisés a priori par des lois de probabilité : il s'agit des paramètres/fonctions techniques ou théoriques $\varepsilon$, $\underline{g}$, $\underline{f}$, $\underline{z}$, $\underline{q}$ et P de la chaîne de traitement biologique 12 et des paramètres de masse moléculaire, $\underline{m}$ et $\underline{\mu}$. Ce sont ces paramètres, parmi lesquels se trouvent les vecteurs $\underline{m}$ et $\underline{\mu}$, qui sont estimés par inversion du modèle direct selon un procédé qui sera détaillé en référence à la figures 3.

Comme illustré sur la figure 2 dans le cadre du premier mode de réalisation, ces paramètres incertains sont définis comme ayant une relation de dépendance probabiliste entre eux, conduisant à un modèle probabiliste global hiérarchique.

Ainsi, dans l'exemple particulier du premier mode de réalisation, le vecteur $\underline{q}$ représentatif des adsorptions est défini comme dépendant du paramètre P de densité des adsorptions. En effet, la variable aléatoire $\underline{q}$ suit une loi de probabilité de Bernoulli qui varie en fonction de la valeur de la variable aléatoire P.

De même, le vecteur $\underline{m}$ représentatif de la masse moléculaire des composants de l'échantillon E est dépendant du vecteur $\underline{q}$ et du paramètre $\underline{\mu}$, comme défini précédemment par la relation $\underline{m} = \underline{\mu} \cdot \underline{q}$.

De même, la fonction $\underline{f}$ représentative de l'évolution temporelle de la fréquence propre de résonance du capteur MEMS ou NEMS, définie par son paramètre $f_0$ et la fonction $c(\underline{m}, \underline{z})$ est dépendante des vecteurs $\underline{m}$ et $\underline{z}$, ce qui correspond à un modèle réaliste du capteur.

De même, la fonction $\underline{g}$ représentative de la sortie du système de lecture 26, définie par son paramètre $g_0$ et la fonction de filtrage $\underline{h}$ réalisant la boucle de lecture, est dépendante de la fonction $\underline{f}$ comme défini précédemment par la relation $\underline{g} = \underline{f} * \underline{h}$.

Enfin, le signal observé $\underline{Y}$ dépend directement de la fonction $\underline{g}$ et du paramètre de bruit $\varepsilon$.

Par conséquent, comme cela est visible sur la figure 2, à un premier niveau de hiérarchie du modèle probabiliste, le signal observé $\underline{Y}$ dépend uniquement des variables aléatoires $\underline{g}$ et $\varepsilon$, cette dernière étant définie par la nature de son modèle probabiliste a priori et la connaissance a priori $\theta_\varepsilon$. A un deuxième niveau de hiérarchie du modèle probabiliste, la variable aléatoire $\underline{g}$ dépend uniquement de la variable aléatoire $\underline{f}$. A un troisième niveau de hiérarchie du modèle probabiliste, la variable aléatoire $\underline{f}$ dépend uniquement des variables aléatoires $\underline{m}$ et $\underline{z}$, cette dernière étant en fait connue grâce à la présence du dispositif de détection 24. A un quatrième niveau de hiérarchie du modèle probabiliste, la variable aléatoire $\underline{m}$ dépend uniquement des variables aléatoires $\underline{q}$ et $\mu$, cette dernière étant définie par la nature de son modèle probabiliste a priori et la connaissance a priori $\theta_m$. Enfin, à un cinquième niveau de hiérarchie du modèle probabiliste, la variable aléatoire $\underline{q}$ dépend uniquement de la variable aléatoire P, cette dernière étant définie par la nature de son modèle probabiliste a priori et la connaissance a priori $\theta_P$. On remarque en particulier que ce modèle hiérarchique engendre, par la dépendance définie entre $\underline{f}$, d'une part, et $\underline{m}$, $\underline{z}$, d'autre part, une hiérarchie entre les paramètres de masse biologique de l'échantillon E et les paramètres techniques de la chaîne de traitement 12 : il présente ainsi en effet un premier étage technique (deuxième et troisième niveaux), dépendant d'un second étage biologique (quatrième et cinquième niveaux), chacun de ces deux étages pouvant lui-même présenter une hiérarchie interne en fonction du modèle retenu.

Sur la base de ce modèle probabiliste hiérarchique, un procédé d'estimation de paramètre de masse moléculaire mis en oeuvre par le processeur 28 par exécution de la séquence d'instructions 30 va maintenant être décrit. Selon une première phase principale, ce procédé réalise une inversion du modèle probabiliste construit au vu du signal observé $\underline{Y}$. Cette inversion permet d'estimer le paramètre de masse $\underline{m}$ des molécules adsorbées sur le capteur MEMS ou NEMS. Selon une seconde phase principale de reconstruction de spectre, ce procédé permet d'estimer le spectre de masse Sp des composants de l'échantillon E à partir du paramètre m̲.

Selon la première phase d'inversion de ce procédé, l'estimation du paramètre m̲ se fait conjointement avec l'estimation de l'ensemble des variables aléatoires ε, g̲, f̲, z̲, m̲, μ, q̲ et P par application d'un estimateur sur la loi de probabilité a posteriori jointe de ces variables aléatoires au vu de l'observation Y̲ ou, de façon équivalente, sur la loi de probabilité jointe de ces variables aléatoires et de l'observation Y̲. En effet, ces deux lois de probabilité sont liées par la règle de Bayes à la probabilité de l'observation Y̲ (ou loi marginale) par la relation suivante :

$$p\left(\underline{Y},\varepsilon,\underline{g},\underline{f},\underline{z},\underline{m},\underline{\mu},\underline{q},P\right) = p\left(\varepsilon,\underline{g},\underline{f},\underline{z},\underline{m},\underline{\mu},\underline{q},P\big|\underline{Y}\right)\cdot p\left(\underline{Y}\right).$$

La loi de probabilité a posteriori jointe se développe en outre de la façon suivante, toujours selon la règle de Bayes :

$$p\left(\varepsilon,\underline{g},\underline{f},\underline{z},\underline{m},\underline{\mu},\underline{q},P\big|\underline{Y}\right) = \frac{p\left(\underline{Y}\big|\varepsilon,\underline{g},\underline{f},\underline{z},\underline{m},\underline{\mu},\underline{q},P\right).p\left(\varepsilon,\underline{g},\underline{f},\underline{z},\underline{m},\underline{\mu},\underline{q},P\right)}{p\left(\underline{Y}\right)}.$$

Bien que la loi de vraisemblance $p(\underline{Y}|\varepsilon,g,f,z,m,\mu,\underline{q},P)$ puisse être exprimée analytiquement et bien que la loi jointe des paramètres $p(\varepsilon, \underline{g}, \underline{f}, \underline{z}, \underline{m}, \underline{\mu}, \underline{q}, P)$ puisse se développer en un produit de lois de probabilités a priori conditionnelles modélisables par l'expérience ou par un calibrage spécifique, la loi marginale $p(\underline{Y})$ n'est pas connue et pas calculable analytiquement. Par conséquent la loi de probabilité a posteriori jointe n'est pas non plus calculable analytiquement puisque l'on ne connaît pas ce facteur multiplicatif $p(\underline{Y})$ qui cependant reste constant pour tous les paramètres. Ce facteur multiplicatif inconnu n'est donc pas pénalisant.

Il en résulte cependant que le calcul d'un estimateur, tel que l'estimateur espérance a posteriori, médiane a posteriori ou maximum a posteriori, sur cette loi a posteriori jointe ne peut se faire analytiquement de façon simple.

De son côté, la loi de probabilité jointe des variables aléatoires et de l'observation se développe de la façon suivante, grâce au modèle hiérarchique de la figure 2 :

$$p\left(\underline{Y},\varepsilon,\underline{g},\underline{f},\underline{z},\underline{m},\underline{\mu},\underline{q},P\right) = \begin{cases} p\left(\underline{Y}\big|\varepsilon,\underline{g}\right)\cdot p\left(\varepsilon\big|\theta_\varepsilon\right)\cdot p\left(\underline{g}\big|\underline{f}\right)\cdot p\left(\underline{f}\big|\underline{z},\underline{m}\right)\cdot p\left(\underline{z}\big|\theta_z\right)\cdot p\left(\underline{m}\big|\underline{\mu},\underline{q}\right)\cdot \\ p\left(\underline{\mu}\big|\theta_m\right)\cdot p\left(\underline{q}\big|P\right)\cdot p\left(P\big|\theta_P\right) \end{cases}$$

Au vu des dépendances déterministes entre certains de ces paramètre et des lois de probabilité a priori choisies pour d'autres (comme indiqué précédemment pour les paramètres ε, μ, q̲ et P), ces connaissances pouvant notamment être acquises par une calibration externe, la loi jointe des variables aléatoires et de l'observation se marginalise aisément en :

$$p\left(\underline{Y},\varepsilon,\underline{\mu},\underline{q},P\right) = \begin{cases} N\left(\underline{Y}\big|c\left(\underline{\mu}\cdot\underline{q},\underline{z}\right)\ast\underline{h},\varepsilon\cdot I_T\right)\cdot \prod_{i=1}^{T} GAM\left(\mu_i\big|k_m,T_m\right)\cdot \left(P^{\Omega(\underline{q},1)}\cdot\left(1-P\right)^{\Omega(\underline{q},0)}\right)\cdot \\ IGAM\left(\varepsilon\big|k_\varepsilon,T_\varepsilon\right)\cdot BET\left(P\big|a_P,b_P\right) \end{cases},$$

où $\Omega,(x,i)$ est une fonction renvoyant le nombre de composantes du vecteur x valant i.

Mais le calcul d'un estimateur, tel que l'estimateur espérance a posteriori, médiane a posteriori ou maximum a posteriori, sur cette loi jointe marginalisée également, ne peut se faire analytiquement de façon simple.

Pour contourner l'impossibilité de calculer directement un tel estimateur sur la loi a posteriori jointe ou la loi jointe marginalisée précitées, il est équivalent et avantageux de procéder à un échantillonnage stochastique numérique de chacun des paramètres inconnus de la loi jointe marginalisée, c'est-à-dire chacun des paramètres ε, μ, q̲ et P, selon la loi de probabilité a posteriori conditionnelle qu'il vérifie, conformément par exemple au procédé connu de Monte-Carlo à chaîne de Markov (procédé d'échantillonnage MCMC, de l'anglais « Markov Chain Monte Carlo »), procédé qui constitue une approximation d'un tirage aléatoire sous la loi a jointe. L'échantillonnage numérique MCMC peut être en particulier réalisé par des méthodes itératives du type échantillonnage stochastique de Gibbs impliquant éventuellement l'algorithme de Metropolis-Hastings (ou équivalent) et l'estimateur, par exemple espérance a posteriori, peut ensuite être approché simplement par les valeurs moyennes des échantillonnages respectifs.

On montre en effet qu'alors que la loi de probabilité a posteriori jointe n'est pas exprimable analytiquement à l'aide de probabilités a priori (conditionnelles ou non), c'est en revanche le cas des lois de probabilité a posteriori conditionnelles précitées, comme cela va maintenant être détaillé.

En particulier, compte tenu de la hiérarchie du modèle probabiliste de la figure 2, compte tenu également de la loi jointe marginalisée détaillée précédemment et de ses paramètres indépendants de $\underline{\mu}$, on montre aisément que la loi de probabilité a posteriori conditionnelle suivie par le paramètre $\underline{\mu}$ prend la forme suivante :

$$p\left(\underline{\mu}\middle|\underline{Y},\varepsilon,\underline{q},P\right) = N\left(\underline{Y}\middle|c\left(\underline{\mu}\cdot\underline{q},\underline{z}\right)*\underline{h},\varepsilon\cdot I_T\right)\cdot\prod_{i=1}^{T}GAM\left(\mu_i\middle|k_m,T_m\right).$$

On montre de la même façon que :

$$p\left(\underline{q}\middle|\underline{Y},\varepsilon,\underline{\mu},P\right) = N\left(\underline{Y}\middle|c\left(\underline{\mu}\cdot\underline{q},\underline{z}\right)*\underline{h},\varepsilon\cdot I_T\right)\cdot\left(P^{\Omega(\underline{q},1)}\cdot\left(1-P\right)^{\Omega(\underline{q},0)}\right),$$

$$\begin{aligned}p\left(\varepsilon\middle|\underline{Y},\underline{\mu},\underline{q},P\right) &= N\left(\underline{Y}\middle|c\left(\underline{\mu}\cdot\underline{q},\underline{z}\right)*\underline{h},\varepsilon\cdot I_T\right)\cdot IGAM\left(\varepsilon\middle|k_\varepsilon,T_\varepsilon\right) \\ &= IGAM\left(\varepsilon\middle|\left(k_\varepsilon+\frac{T}{2}\right),\left(T_\varepsilon+0,5\cdot\left\|\underline{Y}-c\left(\underline{\mu}\cdot\underline{q},\underline{z}\right)*\underline{h}\right\|^2\right)\right),\end{aligned}$$

et

$$\begin{aligned}p\left(P\middle|\underline{Y},\varepsilon,\underline{\mu},\underline{q}\right) &= \left(P^{\Omega(\underline{q},1)}\cdot\left(1-P\right)^{\Omega(\underline{q},0)}\right)\cdot BET\left(P\middle|a_P,b_P\right) \\ &= BET\left(P\middle|\left(a_P+\Omega(\underline{q},1)\right),\left(b_P+\Omega(\underline{q},0)\right)\right).\end{aligned}$$

Les équations ci-dessus montrent que les lois de probabilité a posteriori conditionnelles des paramètres inconnus $\varepsilon$, $\underline{\mu}$, $\underline{q}$ et $P$ sont exprimables analytiquement puisqu'elles sont proportionnelles à des produits de distributions ou probabilités a priori qui sont soit modélisables soit définissables par apprentissage.

Plus précisément, la probabilité a posteriori pour le paramètre de bruit $\varepsilon$ suit une loi gamma-inverse de sorte que l'échantillonnage de ce paramètre pourra se faire simplement et de façon classique dans le cadre d'un procédé itératif d'échantillonnage de Gibbs.

De même, la probabilité a posteriori pour le paramètre $P$ suit une loi bêta de sorte que l'échantillonnage de ce paramètre pourra se faire simplement et de façon classique dans le cadre d'un procédé itératif d'échantillonnage de Gibbs.

En revanche, la probabilité a posteriori pour le paramètre $\underline{\mu}$ s'exprime sous la forme du produit d'une loi normale et de lois gamma. Cette probabilité a posteriori n'a pas d'expression simple, de sorte que l'échantillonnage du paramètre $\underline{\mu}$ ne pourra pas se faire simplement dans le cadre d'un procédé itératif d'échantillonnage de Gibbs. Il sera nécessaire d'utiliser une technique d'échantillonnage à marche aléatoire, telle que l'algorithme de Metropolis-Hastings avec Marche Aléatoire (MHMA) ou un algorithme similaire. Une telle technique d'échantillonnage nécessite de définir à chaque itération un voisinage du paramètre concerné tel qu'estimé à l'itération précédente pour procéder au tirage d'une nouvelle valeur. Le paramètre $\underline{\mu}$ étant par hypothèse un vecteur à valeurs continues, cette notion de voisinage sera classique. Plus précisément, on estime numériquement la loi a posteriori dans un voisinage de la valeur de l'échantillon précédent ; cette loi est normalisée, puis un nouvel échantillon est tiré selon la loi a posteriori ; si cet échantillon n'augmente pas la probabilité jointe, il est accepté avec une probabilité dépendant du rapport de la probabilité jointe avec l'ancien échantillon et avec le nouvel échantillon.

De même, la probabilité a posteriori pour le paramètre $\underline{q}$ s'exprime sous la forme du produit d'une loi normale et d'une loi de Bernoulli. Cette probabilité a posteriori n'a pas d'expression simple non plus, de sorte que l'échantillonnage du paramètre $\underline{q}$ ne pourra pas se faire simplement dans le cadre d'un procédé itératif d'échantillonnage de Gibbs. Il sera nécessaire d'utiliser une technique d'échantillonnage à marche aléatoire, telle que l'algorithme de Metropolis-Hastings avec Marche Aléatoire (MHMA) ou un algorithme approchant, imposant de définir un voisinage du paramètre concerné. Le paramètre $\underline{q}$ étant par hypothèse un vecteur à valeurs binaires, une notion particulière de voisinage va maintenant être définie.

Alors que dans l'état de l'art, certains algorithmes classiques considèrent que le voisinage d'un vecteur est l'ensemble des vecteurs ne s'en différenciant que par une composante, le voisinage d'un vecteur à composantes binaires tel que $q$ est avantageusement défini comme étant constitué des vecteurs binaires :

- tels qu'une adsorption ait été retirée (i.e. une composante à 1 en moins),
- tels qu'une adsorption ait été ajoutée (i.e. une composante à 1 en plus),
- tels qu'une adsorption soit décalée de peu d'échantillons (i.e. une composante à 1 décalée), et
- tels que deux adsorptions aient été regroupées (i.e. deux composantes voisines à 1 regroupées en une seule).

Cette nouvelle définition du voisinage a un impact important sur la bonne convergence de l'échantillonnage. Notamment, elle permet de fortement limiter le risque de converger vers un optimum local.

Au vu de ce qui précède et en référence à la figure 3, un procédé d'estimation de paramètre de masse moléculaire mis en oeuvre par le processeur 28 par exécution de la séquence d'instructions 30 comporte une première phase principale 100 d'estimation conjointe des paramètres dits incertains d'un échantillon biologique E dont on ne connaît pas la composition, dont notamment au moins les paramètres $\mu$ et $q$, par inversion du modèle. Il comporte ensuite une seconde phase principale 200 de reconstruction de spectre de masse à partir de la connaissance de $m = \mu \cdot q$.

L'exécution de la première phase principale d'estimation conjointe 100 et de la seconde phase principale de reconstruction 200 suppose qu'un certain nombre de données et paramètres soient déjà connus et enregistrés dans la base de données 32, à savoir :

- les paramètres dits certains ou constants de la chaîne de traitement biologique 12, c'est-à-dire ceux qui sont invariants d'un traitement biologique à l'autre, et
- les paramètres des modèles de probabilités a priori, conditionnelles ou non, des paramètres incertains à partir desquels peuvent être déterminés les modèles de probabilités a posteriori conditionnelles de chacun des autres paramètres.

Lorsqu'au moins une partie de ces données n'est pas connue, le procédé d'estimation de paramètre de masse moléculaire peut être optionnellement complété d'une phase 300 de calibrage externe précédant les deux phases principales d'estimation conjointe 100 et de reconstruction 200 :

- pour la détermination des paramètres certains non encore connus et leur enregistrement dans la base de données 32, et/ou
- pour la détermination de paramètres stables (par exemple moyenne ou variance) des modèles de probabilités a priori des paramètres incertains et leur enregistrement dans la base de données 32.

La phase principale d'estimation conjointe 100 comporte une première étape de mesure 102 lors de laquelle, conformément au montage de la figure 1, l'échantillon E traverse toute la chaîne de traitement 12 du dispositif 10 pour la fourniture d'un signal observé $Y$.

Ensuite, lors d'une étape d'initialisation 104, les variables aléatoires $\varepsilon$, $\mu$, $q$ et P sont chacune initialisées par le processeur 28 à une première valeur $\varepsilon^{(0)}$, $\mu^{(0)}$, $q^{(0)}$ et $P^{(0)}$. Cette étape peut être réalisée de plusieurs manières :

- initialisation à des valeurs nulles,
- initialisation en échantillonnant les variables avec leurs lois de probabilité a priori, en parcourant le modèle probabiliste de la figure 2 du niveau le plus élevé (à gauche) au niveau le plus bas (à droite), ou
- initialisation avec une méthode peu coûteuse mais moins efficace, telle que par exemple, dériver le signal $Y$ pour initialiser $q$, et garder les N plus faibles valeurs de la dérivée, ...

L'initialisation 104 est également l'étape permettant la pré-allocation de mémoire nécessaire au bon déroulement du procédé.

Le processeur 28 exécute alors, par application d'un algorithme de Monte-Carlo à chaîne de Markov et sur un indice k variant de 1 à kmax, une boucle 106 d'échantillonnage de Gibbs de chacune des variables aléatoires initialisées au vu de leurs lois de probabilité a posteriori conditionnelles respectives telles qu'exprimées analytiquement. kmax est la valeur maximale prise par l'indice k avant qu'un critère d'arrêt prédéterminé ne soit vérifié. Le critère d'arrêt est par exemple un nombre d'itérations maximal fixé a priori, la satisfaction d'un critère de stabilité (tel que le fait qu'une itération supplémentaire n'a pas d'impact significatif sur l'estimateur choisi des variables aléatoires) ou autre.

Plus précisément, pour k variant de 1 à kmax, la boucle 106 comporte les échantillonnages successifs suivants :

- générer un échantillon $\varepsilon^{(k)}$ à partir de la loi a posteriori $p(\varepsilon|Y, \mu^{(k-1)}, q^{(k-1)}, P^{(k-1)})$,

- définir le voisinage du paramètre $\underline{\mu}$ à partir de la valeur $\underline{\mu}^{(k-1)}$,
- générer un échantillon $\underline{\mu}^{(k)}$, dans le voisinage de $\underline{\mu}^{(k-1)}$ à partir de la loi a posteriori $p(\underline{\mu}|\underline{Y},\varepsilon^{(k)},q^{(k-1)},P^{(k-1)})$,
- définir le voisinage du paramètre $\underline{q}$ à partir de la valeur $\underline{q}^{(k-1)}$,
- générer un échantillon $\underline{q}^{(k)}$, dans le voisinage de $\underline{q}^{(k-1)}$ à partir de la loi a posteriori $p(q|\underline{Y},\varepsilon^{(k)},\mu^{(k)},P^{(k-1)})$, et
- générer un échantillon $P^{(k)}$ à partir de la loi a posteriori $p(P|\underline{Y},\varepsilon^{(k)},\underline{\mu}^{(k)},\underline{q}^{(k)})$.

En outre, à chaque itération, la loi jointe marginalisée est estimée et sa valeur est conservée en mémoire avec les valeurs courantes $\varepsilon^{(k)}$, $\underline{\mu}^{(k)}$, $\underline{q}^{(k)}$, $P^{(k)}$.

Le processeur 28 exécute ensuite une étape d'estimation 108 lors de laquelle une estimation est retenue pour chaque paramètre inconnu échantillonné. Chaque paramètre est estimé à partir de l'ensemble des valeurs d'échantillons qu'il a prises et qui ont été prises par la loi jointe marginalisée au fil des itérations.

L'ensemble des valeurs prises en compte peut être réduit aux résultats d'itérations comprises entre un indice kmin et l'indice kmax, kmin étant une valeur de « temps de chauffe » prédéterminée jugée nécessaire pour que, lors de l'échantillonnage de Gibbs, la loi de tirage aléatoire converge vers la loi a posteriori jointe, cette dernière pouvant être également appelée loi cible. Par exemple, pour une boucle de kmax=500 échantillons, une valeur de temps de chauffe de kmin=200 (c'est-à-dire 40% du nombre total d'itérations) semble raisonnable.

Il est ainsi possible, au choix, dans le cas d'un paramètre inconnu à valeurs discrètes (par exemple le paramètre $\underline{q}$) :

- de conserver la valeur de l'échantillon qui a amené à la valeur de la loi jointe marginalisée la plus élevée,
- de moyenner puis de discrétiser les valeurs des N (à définir) échantillons ayant amené les N valeurs les plus élevées de la loi jointe marginalisée,
- de moyenner puis de discrétiser les valeurs des N (à définir, par exemple kmax-kmin) derniers échantillons (estimateur espérance a posteriori),
- de retenir la valeur d'échantillon qui apparaît le plus souvent entre les indices kmin et kmax (estimateur maximum a posteriori),
- etc.

Dans le cas d'un paramètre inconnu à valeurs continues (par exemple le paramètre $\underline{\mu}$), il est possible :

- de conserver la valeur de l'échantillon qui a amené à la valeur de la loi jointe marginalisée la plus élevée,
- de moyenner les valeurs des N (à définir) échantillons ayant amené les N valeurs les plus élevées de la loi jointe marginalisée,
- de moyenner les valeurs des N (à définir, par exemple kmax-kmin) derniers échantillons (estimateur espérance a posteriori),
- de moyenner les valeurs des N (à définir) échantillons ayant amené les N valeurs les plus élevées de la loi jointe et tels que les paramètres inconnus à valeurs discrètes vaillent leur valeur estimée (par exemple, ne moyenner que les valeurs d'échantillons de $\underline{\mu}$ relatifs à des itérations pour lesquelles l'échantillon de $\underline{q}$ prend la valeur de son estimée),
- de moyenner les valeurs des N (à définir) derniers échantillons tels que les paramètres inconnus à valeurs discrètes vaillent leur valeur estimée,
- etc.

L'étape d'estimation 108 fournit ainsi des valeurs estimées $\varepsilon, \underline{\mu}, \hat{\underline{q}}$ et $\hat{P}$ pour chacun des paramètres inconnus $\varepsilon$, $\underline{\mu}$, q et P. On en déduit directement une valeur estimée $\hat{\underline{m}} = \underline{\mu}\cdot\hat{\underline{q}}$ pour le paramètre $\underline{m}$ qui contient les informations de quantité et de masse des molécules adsorbées par le capteur MEMS ou NEMS. Cette dernière étape de la première phase principale d'estimation conjointe 100 est suivie de la seconde phase principale de reconstruction 200.

Au cours d'une première étape 202 de la seconde phase principale de reconstruction 200, les adsorptions détectées mais pour lesquelles la localisation sur le capteur MEMS ou NEMS conduit à une chute de fréquence de

valeur $\dfrac{c(M_i, z_i)}{\underset{z_i}{Max}(c(M_i, z_i))}$ inférieure à un seuil prédéterminé (par exemple 0,25) sont supprimées. Ces suppressions sont qualifiées de « rejets en z ». Le taux de rejets en z $TR_Z$ est également calculé à cette étape.

Au cours d'une étape 204 suivante, un histogramme du paramètre $\underline{m}$ est calculé, ce dernier correspondant au spectre de masse de l'échantillon E. Cette étape de calcul est classique et ne sera pas détaillée.

Enfin, au cours d'une étape 206, cet histogramme est multiplié par une constante qui s'avère être l'inverse d'un gain, en particulier l'inverse du gain global de la chaîne de traitement 12, à savoir le produit Ge.Gf.Gc, multiplié par l'inverse de $(1-TR_z)$ (complément à 1 du taux de rejets en z calculé à l'étape 202). On obtient ainsi le spectre de masse Sp des composants de l'échantillon E, qui est, comme le paramètre $\underline{m}$, un paramètre de masse moléculaire caractéristique du profil moléculaire de cet échantillon.

En variante du premier mode de réalisation qui vient d'être décrit, un calibrage interne peut être réalisé en incorporant dans l'échantillon E, à l'étape 102, un échantillon E* de composants connus alourdis de marquage.

Une première conséquence d'un tel calibrage interne réalisé pendant l'étape de mesure 102 est d'ajouter des connaissances a priori sur au moins une partie des paramètres de la chaîne de traitement 12. En particulier, les composantes $\mu_k$ du paramètre vectoriel $\mu$ étant modélisées selon une loi gamma $GAM(\mu_k|k_m, T_m)$, la connaissance a priori $\theta_m$ sur ce paramètre peut être plus concentrée autour des masses alourdies introduites.

Une autre conséquence concerne l'étape 206, l'inverse du gain utilisé pour pondérer l'histogramme du paramètre $\underline{m}$ n'étant pas connu a priori mais estimé par le dispositif de traitement 14 grâce au calibrage interne.

La phase de calibrage externe 300 de la chaîne de traitement biologique 12 comporte une première étape de mesure 302 lors de laquelle, conformément au montage de la figure 1, l'échantillon de protéines de calibrage externe $E_{CALIB}$ traverse toute la chaîne de traitement 12 du dispositif 10 pour la fourniture d'un signal observé $\underline{Y}_{CALIB}$.

Le traitement appliqué par le processeur 28 au signal $\underline{Y}_{CALIB}$ consiste à déterminer des valeurs non encore connues de paramètres certains, c'est-à-dire des paramètres qui restent en réalité relativement constants d'un traitement biologique à l'autre. Ces paramètres certains sont alors considérés et modélisés par des constantes dans la chaîne de traitement biologique 12. Il s'agit par exemple des différents gains de la chaîne de traitement 12 tels que Ge, Gf et Gc, des paramètres $f_0$ ou $g_0$ précités, de la fonction de filtrage $\underline{h}$ de la boucle de lecture, etc. Ce traitement peut consister également à déterminer des paramètres stables (par exemple moyenne ou variance) des modèles de probabilités a priori de paramètres incertains tels que ceux précités. Ces paramètres stables sont alors également considérés et modélisés par des constantes.

Dans l'échantillon de protéines de calibrage externe, certains des paramètres incertains sont cette fois-ci connus comme le paramètre de masse moléculaire $\underline{m}$ ou le spectre de masse Sp, mais comme pour la première phase principale 100, la détermination se fait par application d'un échantillonnage numérique conforme au procédé de Monte-Carlo à chaîne de Markov. Elle se fait cette fois-ci cependant de façon classique sur les paramètres certains non connus et est illustrée par la référence 304. L'étape 304 reproduit donc une partie des étapes de détermination 104, 106 et 108 de la phase principale 100.

Enfin, lors d'une dernière étape 306 de la première phase de calibrage externe, les paramètres certains et stables déterminés à l'étape précédente sont enregistrés dans la base de données 32.

Si, au cours de cette calibration externe, plusieurs molécules de concentrations connues sont introduites, il faut calculer les paramètres certains et stables (parmi lesquels les constantes de gains) pour chacun des types de molécules. Ensuite, il suffit par exemple de retenir la moyenne de ces calculs, leur médiane, ou la moyenne des valeurs les moins extrêmes. Cette méthode peut également être appliquée en calibration interne, le cas échéant.

Dans un deuxième mode de réalisation, dans lequel on suppose que les molécules de l'échantillon présentent toutes une même et unique masse $M_0$, le paramètre $\underline{m}$ peut être affiné en introduisant le paramètre théorique $\underline{q}$ mentionné dans le premier mode de réalisation. Il peut alors s'exprimer sous la forme $\underline{m} = M_0 \cdot \underline{q}$ où $M_0$ est une constante.

Dans ce nouveau modèle choisi, le paramètre $M_0$ est incertain et est modélisé selon une loi de probabilité a priori prédéterminée, par exemple une loi gamma $GAM(M_0|k_m, T_m)$ de forme $k_m$ et d'intensité $T_m$, ces deux derniers paramètres représentant la connaissance a priori sur le paramètre de masse moléculaire notée $\theta_m$.

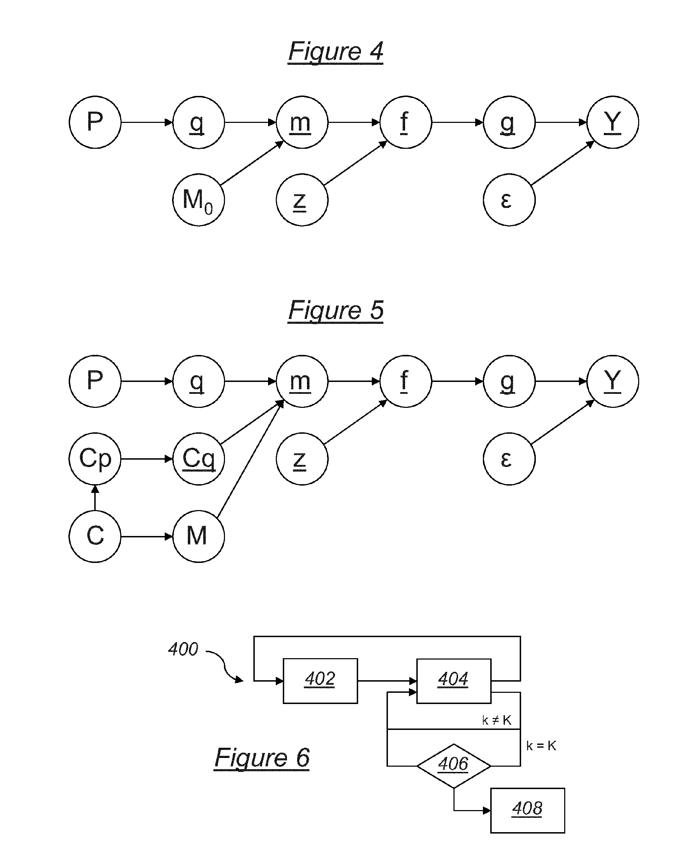

Comme illustré sur la figure 4 dans le cadre du deuxième mode de réalisation, les paramètres incertains du nouveau modèle, tous identiques à ceux du modèle précédent à l'exception de $M_0$, sont définis comme ayant une relation de dépendance probabiliste entre eux, conduisant à un modèle probabiliste global hiérarchique.

Ainsi, le paramètre $\underline{q}$ est dépendant du paramètre P, le paramètre $\underline{m}$ dépendant du paramètre $\underline{q}$ et du paramètre $M_0$, comme défini précédemment par la relation $\underline{m} = M_0 \cdot \underline{q}$, la fonction $\underline{f}$ dépendante des paramètres $\underline{m}$ et $\underline{z}$, la fonction $\underline{g}$ dépendante de la fonction $\underline{f}$ et le signal observé $\underline{Y}$ dépendant de la fonction $\underline{g}$ et du paramètre de bruit $\varepsilon$.

Par conséquent, comme cela est visible sur la figure 4, à un premier niveau de hiérarchie du modèle probabiliste, le signal observé $\underline{Y}$ dépend uniquement des variables aléatoires $\underline{g}$ et $\varepsilon$, cette dernière étant définie par la nature de son modèle probabiliste a priori et la connaissance a priori $\theta_\varepsilon$. A un deuxième niveau de hiérarchie du modèle probabiliste, la variable aléatoire $\underline{g}$ dépend uniquement de la variable aléatoire $\underline{f}$. A un troisième niveau de hiérarchie du modèle probabiliste, la variable aléatoire $\underline{f}$ dépend uniquement des variables aléatoires $\underline{m}$ et $\underline{z}$, cette dernière étant en fait connue grâce à la présence du dispositif de détection 24. A un quatrième niveau de hiérarchie du modèle probabiliste, la variable aléatoire $\underline{m}$ dépend uniquement des variables aléatoires $\underline{q}$ et $M_0$, cette dernière étant définie par la nature de son modèle probabiliste a priori et la connaissance a priori $\theta_m$. Enfin, à un cinquième niveau de hiérarchie du modèle probabiliste, la variable aléatoire $\underline{q}$ dépend uniquement de la variable aléatoire P, cette dernière étant définie par la nature de son modèle probabiliste a priori et la connaissance a priori $\theta_P$.

Sur la base de ce modèle probabiliste hiérarchique, le procédé d'estimation de paramètre de masse moléculaire décrit précédemment s'applique de la même façon pour une estimation du paramètre $\underline{m}$ et du spectre de masse Sp.

Selon la première phase d'inversion de ce procédé, l'estimation du paramètre $\underline{m}$ se fait conjointement avec l'estimation de l'ensemble des variables aléatoires $\varepsilon$, $\underline{g}$, $\underline{f}$, $\underline{z}$, $\underline{m}$, $M_0$, $\underline{q}$ et $P$ par application d'un estimateur sur la loi de probabilité a posteriori jointe de ces variables aléatoires au vu de l'observation $\underline{Y}$ ou, de façon équivalente, sur la loi de probabilité jointe de ces variables aléatoires et de l'observation $\underline{Y}$.

En particulier, la loi de probabilité jointe des variables aléatoires et de l'observation se développe de la façon suivante, grâce au modèle hiérarchique de la figure 4 :

$$p\left(\underline{Y},\varepsilon,\underline{g},\underline{f},\underline{z},\underline{m},M_0,\underline{q},P\right)=\begin{cases} p\left(\underline{Y}\middle|\varepsilon,\underline{g}\right)\cdot\ p\left(\varepsilon\middle|\theta_\varepsilon\right)\cdot\ p\left(\underline{g}\middle|\underline{f}\right)\cdot\ p\left(\underline{f}\middle|\underline{z},\underline{m}\right)\cdot\ p\left(\underline{z}\middle|\theta_z\right)\cdot \\ p\left(\underline{m}\middle|M_0,\underline{q}\right)\cdot\ p\left(M_0\middle|\theta_m\right)\cdot\ p\left(\underline{q}\middle|P\right)\cdot\ p\left(P\middle|\theta_P\right) \end{cases}.$$

Au vu des dépendances déterministes entre certains de ces paramètre et des lois de probabilité a priori choisies pour d'autres (comme indiqué précédemment pour les paramètres $\varepsilon$, $M_0$, $\underline{q}$ et $P$), ces connaissances pouvant notamment être acquises par une calibration externe, la loi jointe des variables aléatoires et de l'observation se marginalise aisément en :

$$p\left(\underline{Y},\varepsilon,M_0,\underline{q},P\right)=\begin{cases} N\left(\underline{Y}\middle|c\left(M_0\cdot\underline{q},\underline{z}\right)*\underline{h},\varepsilon\cdot I_T\right)\cdot GAM\left(M_0\middle|k_m,T_m\right)\cdot\left(P^{\Omega(\underline{q},1)}\cdot\left(1-P\right)^{\Omega(\underline{q},0)}\right)\cdot \\ IGAM\left(\varepsilon\middle|k_\varepsilon,T_\varepsilon\right)\cdot BET\left(P\middle|a_P,b_P\right) \end{cases}.$$

Compte tenu de la hiérarchie du modèle probabiliste de la figure 4, compte tenu également de la loi jointe marginalisée ci-dessus et de ses paramètres indépendants de $M_0$, on montre aisément que la loi de probabilité a posteriori conditionnelle suivie par le paramètre $M_0$ prend la forme suivante :

$$p\left(M_0\middle|\underline{Y},\varepsilon,\underline{q},P\right)=N\left(\underline{Y}\middle|c\left(M_0\cdot\underline{q},\underline{z}\right)*\underline{h},\varepsilon\cdot I_T\right)\cdot GAM\left(M_0\middle|k_m,T_m\right).$$

On montre de la même façon que :

$$p\left(\underline{q}\middle|\underline{Y},\varepsilon,M_0,P\right)=N\left(\underline{Y}\middle|c\left(M_0\cdot\underline{q},\underline{z}\right)*\underline{h},\varepsilon\cdot I_T\right)\cdot\left(P^{\Omega(\underline{q},1)}\cdot\left(1-P\right)^{\Omega(\underline{q},0)}\right),$$

$$\begin{aligned} p\left(\varepsilon\middle|\underline{Y},M_0,\underline{q},P\right) &= N\left(\underline{Y}\middle|c\left(M_0\cdot\underline{q},\underline{z}\right)*\underline{h},\varepsilon\cdot I_T\right)\cdot IGAM\left(\varepsilon\middle|k_\varepsilon,T_\varepsilon\right) \\ &= IGAM\left(\varepsilon\middle|\left(k_\varepsilon+\frac{T}{2}\right),\left(T_\varepsilon+0,5\cdot\left\|\underline{Y}-c\left(M_0\cdot\underline{q},\underline{z}\right)*\underline{h}\right\|^2\right)\right), \end{aligned}$$

et

$$\begin{aligned} p\left(P\middle|\underline{Y},\varepsilon,M_0,\underline{q}\right) &= \left(P^{\Omega(\underline{q},1)}\cdot\left(1-P\right)^{\Omega(\underline{q},0)}\right)\cdot BET\left(P\middle|a_P,b_P\right) \\ &= BET\left(P\middle|\left(a_P+\Omega(\underline{q},1)\right),\left(b_P+\Omega(\underline{q},0)\right)\right). \end{aligned}$$

Les équations ci-dessus montrent que les lois de probabilité a posteriori conditionnelles des paramètres inconnus $\varepsilon$, $M_0$, $\underline{q}$ et $P$ sont exprimables analytiquement et exploitables de la même façon que dans le premier mode de réalisation.

Au vu de ce qui précède, le procédé d'estimation de paramètre de masse moléculaire illustré sur la figure 3, incluant la première phase principale d'estimation conjointe 100, la seconde phase principale de reconstruction 200 et l'éventuelle phase préalable de calibrage externe 300, s'applique de la même façon.

En particulier, lors de l'étape d'initialisation 104, les variables aléatoires $\varepsilon$, $M_0$, $\underline{q}$ et P sont chacune initialisées par le processeur 28 à une première valeur $\varepsilon^{(0)}$, $M_0^{(0)}$, $\underline{q}^{(0)}$ et $P^{(0)}$.

Le processeur 28 exécute alors, par application d'un algorithme de Monte-Carlo à chaîne de Markov et sur un indice k variant de 1 à kmax, la boucle 106 d'échantillonnage de Gibbs de chacune des variables aléatoires initialisées au vu de leurs lois de probabilité a posteriori conditionnelles respectives telles qu'exprimées analytiquement.

Plus précisément, pour k variant de 1 à kmax, la boucle 106 comporte les échantillonnages successifs suivants :

- générer un échantillon $\varepsilon^{(k)}$ à partir de la loi a posteriori $p(\varepsilon|\underline{Y},M_0^{(k-1)},\underline{q}^{(k-1)},P^{(k-1)})$,
- définir le voisinage du paramètre $M_0$ à partir de la valeur $M_0^{(k-1)}$,
- générer un échantillon $M_0^{(k)}$, dans le voisinage de $M_0^{(k-1)}$ à partir de la loi a posteriori $p(M_0|\underline{Y},\varepsilon^{(k)}, \underline{q}^{(k-1)},P^{(k-1)})$,
- définir le voisinage du paramètre $\underline{q}$ à partir de la valeur $\underline{q}^{(k-1)}$,
- générer un échantillon $\underline{q}^{(k)}$, dans le voisinage de $\underline{q}^{(k-1)}$ à partir de la loi a posteriori $p(\underline{q}|\underline{Y},\varepsilon^{(k)},M_0^{(k)},P^{(k-1)})$, et
- générer un échantillon $P^{(k)}$ à partir de la loi a posteriori $p(P|\underline{Y},\varepsilon^{(k)},M_0^{(k)},\underline{q}^{(k)})$.

En outre, à chaque itération, la loi jointe marginalisée est estimée et sa valeur est conservée en mémoire avec les valeurs courantes $\varepsilon^{(k)}$, $M_0^{(k)}$, $\underline{q}^{(k)}$, $P^{(k)}$.

L'étape d'estimation 108 fournit des valeurs estimées $\hat{\varepsilon}, \hat{M}_0, \hat{\underline{q}}$ et $P$ pour chacun des paramètres inconnus $\varepsilon$, $M_0$, $\underline{q}$ et P. On en déduit directement une valeur estimée $\underline{m} = \hat{M}_0 \cdot \hat{\underline{q}}$ pour le paramètre $\underline{m}$.

La phase suivante de reconstruction de spectre 200 est également inchangée.

On notera enfin qu'une variante avec calibrage interne ne peut pas être réalisée dans ce deuxième mode de réalisation puisqu'il est pris pour hypothèse que l'échantillon analysé ne comporte que des composants présentant une même et unique masse moléculaire. Un tel calibrage ramènerait le modèle à celui d'un troisième mode de réalisation qui va maintenant être détaillé.

Dans ce troisième mode de réalisation, dans lequel on suppose que les molécules de l'échantillon E présentent une pluralité discrète de masses moléculaires, le paramètre $\underline{m}$ peut être affiné en introduisant le paramètre théorique $\underline{q}$ mentionné dans les deux premiers modes de réalisation. A la différence d'une distribution continue de masses, on suppose que les molécules de l'échantillon E* sont regroupées en classes, chaque molécule d'une classe donnée ayant la même masse que toutes les autres molécules de la même classe. On note C le nombre de ces classes. Le paramètre $\underline{m}$ peut alors s'exprimer sous la forme $\underline{m} = M(C_q) \cdot \underline{q}$ où $\underline{C_q}$ est un vecteur à T composantes, chacune de ses composantes indiquant l'indice i, $1 \leq i \leq C$, d'une classe et où $M(\underline{C_q})$ est un vecteur à T composantes, chacune de ses composantes indiquant la masse associée à la classe indiquée dans la composante correspondante du vecteur $\underline{C_q}$. Pour passer du vecteur $\underline{C_q}$ de succession de classes au vecteur $M(\underline{C_q})$ de succession de masses, on introduit un paramètre vectoriel M à C composantes indépendantes correspondant respectivement aux C classes, chacune de ses composantes indiquant la masse associée à la classe correspondante.

Il est également nécessaire d'introduire un autre paramètre Cp, vecteur à C composantes, chaque composante de ce vecteur indiquant la probabilité de la classe correspondante, le vecteur indiquant donc au final la répartition des C classes en termes de probabilité.

Dans ce nouveau modèle choisi, le paramètre vectoriel M est incertain et ses C composantes indépendantes $M_i$, $1 \leq i \leq C$, sont modélisées selon une loi de probabilité a priori prédéterminée, par exemple une loi gamma $GAM(M_i|k_m,T_m)$ de forme $k_m$ et d'intensité $T_m$, ces deux derniers paramètres représentant la connaissance a priori sur le paramètre de masse moléculaire notée $\theta_m$.

Dans ce nouveau modèle choisi également, le paramètre entier C est incertain et est modélisé selon une loi de probabilité a priori prédéterminée, par exemple une loi de Poisson de paramètre $\lambda_C$, ce paramètres représentant la connaissance a priori sur le paramètre C notée $\theta_C$. En variante, le paramètre C pourrait être connu et sa loi de probabilité a priori représentée par une distribution de Dirac centrée sur la valeur connue.

Dans ce nouveau modèle choisi également, le paramètre vectoriel $\underline{C_q}$ est incertain, ses composantes sont à valeurs discrètes comprises entre 1 et C et la probabilité pour que l'une de ses composantes quelconque vaille i, $1 \leq i \leq C$, vaut la valeur de la i-ème composante de Cp.

Dans ce nouveau modèle choisi également, le paramètre vectoriel Cp est incertain et est modélisé selon une loi de probabilité a priori prédéterminée, par exemple une loi de Dirichlet $D(C_p,\{a_{Cp},...,a_{Cp}\})$ de paramètres de forme tous égaux à une valeur $a_{Cp}$, cette valeur commune représentant la connaissance a priori sur le paramètre Cp notée $\theta_{Cp}$.

Comme illustré sur la figure 5 dans le cadre du troisième mode de réalisation, les paramètres incertains du nouveau modèle sont définis comme ayant une relation de dépendance probabiliste entre eux, conduisant à un modèle probabiliste global hiérarchique.

Ainsi, le paramètre q est dépendant du paramètre P, le paramètre $\underline{m}$ dépendant du paramètre q, du paramètre $\underline{C_q}$ et du paramètre M comme défini précédemment par la relation $\underline{m} = M(\underline{C_q}) \cdot \underline{q}$, le paramètre $\underline{C_q}$ dépendant du paramètre Cp par définition, les paramètres M et Cp dépendants du paramètre C qui définit leur taille vectorielle, la

fonction $\underline{f}$ dépendante des paramètres $\underline{m}$ et $\underline{z}$, la fonction $\underline{g}$ dépendante de la fonction $\underline{f}$ et le signal observé $\underline{Y}$ dépendant de la fonction $\underline{g}$ et du paramètre de bruit $\varepsilon$.

Par conséquent, comme cela est visible sur la figure 5, à un premier niveau de hiérarchie du modèle probabiliste, le signal observé $\underline{Y}$ dépend uniquement des variables aléatoires $\underline{g}$ et $\varepsilon$, cette dernière étant définie par la nature de son modèle probabiliste a priori et la connaissance a priori $\theta_\varepsilon$. A un deuxième niveau de hiérarchie du modèle probabiliste, la variable aléatoire $\underline{g}$ dépend uniquement de la variable aléatoire $\underline{f}$. A un troisième niveau de hiérarchie du modèle probabiliste, la variable aléatoire $\underline{f}$ dépend uniquement des variables aléatoires $\underline{m}$ et $\underline{z}$, cette dernière étant en fait connue grâce à la présence du dispositif de détection 24. A un quatrième niveau de hiérarchie du modèle probabiliste, la variable aléatoire $\underline{m}$ dépend uniquement des variables aléatoires $\underline{q}$, $\underline{Cq}$ et M, cette dernière étant définie par la nature de son modèle probabiliste a priori et la connaissance a priori $\theta_m$. A un cinquième niveau de hiérarchie du modèle probabiliste, la variable aléatoire $\underline{q}$ dépend uniquement de la variable aléatoire P, cette dernière étant définie par la nature de son modèle probabiliste a priori et la connaissance a priori $\theta_P$. Au cinquième niveau de hiérarchie du modèle probabiliste également, la variable aléatoire $\underline{Cq}$ dépend uniquement de la variable aléatoire Cp, cette dernière étant définie par la nature de son modèle probabiliste a priori et la connaissance a priori $\theta_{Cp}$. Au cinquième niveau de hiérarchie du modèle probabiliste également, la variable aléatoire M dépend uniquement de la variable aléatoire C, cette dernière étant définie par la nature de son modèle probabiliste a priori et la connaissance a priori $\theta_C$. Enfin, à un sixième niveau de hiérarchie du modèle probabiliste également, la variable aléatoire Cp dépend uniquement de la variable aléatoire C.

Sur la base de ce modèle probabiliste hiérarchique, le procédé d'estimation de paramètre de masse moléculaire décrit précédemment s'applique de façon similaire pour une estimation du paramètre $\underline{m}$ et du spectre de masse Sp.

Selon la première phase d'inversion de ce procédé, l'estimation du paramètre $\underline{m}$ se fait conjointement avec l'estimation de l'ensemble des variables aléatoires $\varepsilon$, $\underline{g}$, $\underline{f}$, $\underline{z}$, $\underline{m}$, M, $\underline{Cq}$, Cp, C, $\underline{q}$ et P par application d'un estimateur sur la loi de probabilité a posteriori jointe de ces variables aléatoires au vu de l'observation $\underline{Y}$ ou, de façon équivalente, sur la loi de probabilité jointe de ces variables aléatoires et de l'observation $\underline{Y}$.

En particulier, la loi de probabilité jointe des variables aléatoires et de l'observation se développe de la façon suivante, grâce au modèle hiérarchique de la figure 5 :

$$p\left(\underline{Y},\varepsilon,\underline{g},\underline{f},\underline{z},\underline{m},\underline{q},P,M,\underline{Cq},Cp,C\right)=\begin{cases} p\left(\underline{Y}|\varepsilon,\underline{g}\right)\cdot p\left(\varepsilon|\theta_\varepsilon\right)\cdot p\left(\underline{g}|\underline{f}\right)\cdot p\left(\underline{f}|\underline{z},\underline{m}\right)\cdot p\left(\underline{z}|\theta_z\right)\cdot \\ p\left(\underline{m}|M,\underline{Cq},\underline{q}\right)\cdot p\left(\underline{q}|P\right)\cdot p\left(P|\theta_P\right)\cdot p\left(\underline{Cq}|Cp\right)\cdot \\ p\left(M|\theta_m,C\right)\cdot p\left(Cp|\theta_{Cp},C\right)\cdot p\left(C|\theta_C\right) \end{cases}$$

Au vu des dépendances déterministes entre certains de ces paramètre et des lois de probabilité a priori choisies pour d'autres (comme indiqué précédemment pour les paramètres $\varepsilon$, M, $\underline{q}$, P, $\underline{Cq}$, Cp, C), ces connaissances pouvant notamment être acquises par une calibration externe, la loi jointe des variables aléatoires et de l'observation se marginalise aisément en :

$$p\left(\underline{Y},\varepsilon,M,\underline{q},P,\underline{Cq},Cp,C\right)=\begin{cases} N\left(\underline{Y}|c\left(M\left(\underline{Cq}\right)\cdot\underline{q},\underline{z}\right)*\underline{h},\varepsilon\cdot I_T\right)\cdot IGAM\left(\varepsilon|k_\varepsilon,T_\varepsilon\right)\cdot \\ \prod_{i=1}^{C} GAM\left(M_i|k_m,T_m\right)\cdot\left(P^{\Omega(\underline{q},1)}\cdot\left(1-P\right)^{\Omega(\underline{q},0)}\right)\cdot \\ BET\left(P|a_P,b_P\right)\cdot\prod_{i=1}^{C} Cp_i^{\Omega(\underline{Cq},i)}\cdot D\left(Cp,a_{Cp},...,a_{Cp}\right)\cdot e^{-\lambda_C}\cdot\dfrac{\lambda_C}{C!} \end{cases}.$$

Compte tenu de la hiérarchie du modèle probabiliste de la figure 5, compte tenu également de la loi jointe marginalisée ci-dessus et de ses paramètres indépendants de M, on montre aisément que la loi de probabilité a posteriori conditionnelle suivie par le paramètre M prend la forme suivante :

$$p\left(M|\underline{Y},\varepsilon,\underline{q},P,\underline{Cq},Cp,C\right)=N\left(\underline{Y}|c\left(M\left(\underline{Cq}\right)\cdot\underline{q},\underline{z}\right)*\underline{h},\varepsilon\cdot I_T\right)\cdot\prod_{i=1}^{C} GAM\left(M_i|k_m,T_m\right).$$

On montre de la même façon que :

$$p\left(\underline{q}\middle|\underline{Y},\varepsilon,M,P,\underline{Cq},Cp,C\right)=N\left(\underline{Y}\middle|c\left(M\left(\underline{Cq}\right)\cdot\underline{q},\underline{z}\right)*\underline{h},\varepsilon\cdot I_T\right)\cdot\left(P^{\Omega\left(\underline{q},1\right)}\cdot\left(1-P\right)^{\Omega\left(\underline{q},0\right)}\right),$$

$$p\left(\varepsilon\middle|\underline{Y},M,\underline{q},P,\underline{Cq},Cp,C\right)=N\left(\underline{Y}\middle|c\left(M\left(\underline{Cq}\right)\cdot\underline{q},\underline{z}\right)*\underline{h},\varepsilon\cdot I_T\right)\cdot IGAM\left(\varepsilon\middle|k_\varepsilon,T_\varepsilon\right)$$

$$=IGAM\left(\varepsilon\middle|\left(k_\varepsilon+\frac{T}{2}\right),\left(T_\varepsilon+0,5\cdot\left\|\underline{Y}-c\left(M\left(\underline{Cq}\right)\cdot\underline{q},\underline{z}\right)*\underline{h}\right\|^2\right)\right),$$

$$p\left(P\middle|\underline{Y},\varepsilon,M,\underline{q},\underline{Cq},Cp,C\right)=\left(P^{\Omega\left(\underline{q},1\right)}\cdot\left(1-P\right)^{\Omega\left(\underline{q},0\right)}\right)\cdot BET\left(P\middle|a_P,b_P\right)$$

$$=BET\left(P\middle|\left(a_P+\Omega\left(\underline{q},1\right)\right),\left(b_P+\Omega\left(\underline{q},0\right)\right)\right),$$

$$p\left(\underline{Cq}\middle|\underline{Y},\varepsilon,M,\underline{q},P,Cp,C\right)=N\left(\underline{Y}\middle|c\left(M\left(\underline{Cq}\right)\cdot\underline{q},\underline{z}\right)*\underline{h},\varepsilon\cdot I_T\right)\cdot\prod_{i=1}^{C}Cp_i^{\Omega\left(\underline{Cq},i\right)},$$

$$p\left(Cp\middle|\underline{Y},\varepsilon,M,\underline{q},P,\underline{Cq},C\right)=\prod_{i=1}^{T}Cq_i^{\Omega\left(\underline{Cq},i\right)}\cdot D\left(Cp,a_{Cp},...,a_{Cp}\right)$$

$$=D\left(Cp,\left(a_{Cp}+\Omega\left(\underline{q},1\right)\right),...,\left(a_{Cp}+\Omega\left(\underline{q},C\right)\right)\right)$$

Les équations ci-dessus montrent que les lois de probabilité a posteriori conditionnelles des paramètres inconnus $\varepsilon$, M, $\underline{q}$, P, $\underline{Cq}$ et Cp sont exprimables analytiquement puisqu'elles sont proportionnelles à des produits de distributions ou probabilités a priori qui sont soit modélisables soit définissables par apprentissage.

Plus précisément, la probabilité a posteriori pour le paramètre de bruit $\varepsilon$ suit une loi gamma-inverse, comme dans les modes de réalisation précédents, de sorte que l'échantillonnage de ce paramètre pourra se faire simplement et de façon classique dans le cadre d'un procédé itératif d'échantillonnage de Gibbs.

De même, la probabilité a posteriori pour le paramètre P suit une loi bêta, comme dans les modes de réalisation précédents, de sorte que l'échantillonnage de ce paramètre pourra se faire simplement et de façon classique dans le cadre d'un procédé itératif d'échantillonnage de Gibbs.

La probabilité a posteriori pour le paramètre M s'exprime sous la forme du produit d'une loi normale et de lois gamma de sorte qu'il sera nécessaire d'utiliser une technique d'échantillonnage à marche aléatoire, comme dans les modes de réalisation précédents pour $\underline{\mu}$ et $M_0$. Une telle technique d'échantillonnage nécessite de définir à chaque itération un voisinage du paramètre M tel qu'estimé à l'itération précédente pour procéder au tirage d'une nouvelle valeur. Ce voisinage sera défini comme dans les modes de réalisation précédents pour $\underline{\mu}$ et $M_0$.

La probabilité a posteriori pour le paramètre $\underline{q}$ s'exprime sous la forme du produit d'une loi normale et d'une loi de Bernoulli de sorte qu'il sera nécessaire d'utiliser une technique d'échantillonnage à marche aléatoire, comme dans les modes de réalisation précédents. Une telle technique d'échantillonnage nécessite de définir à chaque itération un voisinage du paramètre $\underline{q}$ tel qu'estimé à l'itération précédente pour procéder au tirage d'une nouvelle valeur. Ce voisinage sera défini comme dans les modes de réalisation précédents.

La probabilité a posteriori pour le paramètre $\underline{Cq}$ s'exprime sous la forme du produit d'une loi normale et d'un nombre variable de probabilités a priori. Cette probabilité a posteriori n'a pas d'expression simple, de sorte qu'il sera nécessaire d'utiliser une technique d'échantillonnage à marche aléatoire, telle que l'algorithme de Metropolis-Hastings avec Marche Aléatoire (MHMA) ou un algorithme approchant, imposant de définir un voisinage du paramètre $\underline{Cq}$. Ce paramètre étant par hypothèse un vecteur à valeurs discrètes, une notion particulière de voisinage va maintenant être définie.

Selon une première variante, on itère sur les composantes du vecteur $\underline{Cq}$ un processus qui propose à chaque itération autant de voisins qu'il y a de classes. Ainsi, à la k-ième itération de ce processus, la k-ième composante de $\underline{Cq}$ pourra valoir tous les entiers entre 1 et C : elle aura C-1 voisins sélectionnables (en plus, éventuellement, de la valeur courante) lors de l'échantillonnage.

Selon une seconde variante, avantageuse pour gagner du temps de calcul en cas de nombre élevé de classes, il suffit de définir un seul voisin pour la k-ième composante de Cq, tiré au hasard parmi les C-1 valeurs possibles autres que la valeur courante : la k-ième composante aura donc 1 voisin sélectionnable en plus de la valeur courante lors de l'échantillonnage.

La probabilité a posteriori pour le paramètre Cp suit une loi de Dirichlet de sorte que l'échantillonnage de ce paramètre pourra se faire simplement et de façon classique dans le cadre d'un procédé itératif d'échantillonnage de Gibbs.

En revanche, il n'y a pas d'expression analytique exploitable de la probabilité a posteriori pour le paramètre C de sorte que son échantillonnage doit s'effectuer selon une méthode particulière, en coordination avec l'échantillonnage des autres paramètres inconnus.

Au vu de ce qui précède, le procédé d'estimation de paramètre de masse moléculaire illustré sur la figure 3, incluant la première phase principale d'estimation conjointe 100, la seconde phase principale de reconstruction 200 et l'éventuelle phase préalable de calibrage externe 300, s'applique de la même façon, sauf pour la boucle d'échantillonnage de Gibbs 106 qui doit être remplacée par une boucle d'échantillonnage 400 plus complexe, adaptée à l'échantillonnage particulier du paramètre C et illustrée sur la figure 6.

Ainsi, après avoir initialisé chacune des variables aléatoires $\varepsilon$, M, $\underline{q}$, P, $\underline{Cq}$, Cp et C à une première valeur $\theta^{(0)}$, $M^{(0)}$, $\underline{q}^{(0)}$, $P^{(0)}$, $\underline{Cq}^{(0)}$, $Cp^{(0)}$ et $C^{(0)}$ lors de l'étape d'initialisation 104 et après avoir initialisé la valeur de la loi jointe marginalisée, le processeur 28 exécute la boucle d'échantillonnage 400.

Au cours d'une première étape 402 de cette boucle d'échantillonnage 400, on génère un échantillon de C selon sa loi de probabilité a priori autour de sa valeur courante, les dernières valeurs des autres paramètres inconnus et la dernière valeur de la loi jointe marginalisée étant stockées en mémoire.

Si cette loi de probabilité a priori est une loi de Poisson comme indiqué précédemment, l'échantillonnage 402 du paramètre C consiste à ajouter ou retirer une classe et :

- si une classe est retirée, à fusionner les deux classes les plus proches en termes de masses,
- si une classe est ajoutée, à créer une nouvelle classe dont la masse est tirée selon la loi a priori du paramètre M.

On notera qu'il est possible, de façon optionnelle, de favoriser (respectivement pénaliser) le retrait d'une classe s'il existe (respectivement s'il n'existe pas) deux classes très proches en terme de masse.

Ensuite, au cours d'une étape d'échantillonnage de Gibbs 404, les autres paramètres $\varepsilon$, M, $\underline{q}$, P, $\underline{Cq}$ et Cp sont échantillonnés.

Plus précisément, pour k variant de 1 à au plus kmax, l'étape 404 comporte les échantillonnages successifs suivants :

- générer un échantillon $\varepsilon^{(k)}$ à partir de la loi a posteriori $p(\varepsilon|\underline{Y},\underline{q}^{(k-1)}, P^{(k-1)}, M^{(k-1)}, \underline{Cq}^{(k-1)}, C_p^{(k-1)})$,
- définir le voisinage du paramètre $\underline{q}$ à partir de la valeur $\underline{q}^{(k-1)}$,
- générer un échantillon $\underline{q}^{(k)}$ dans le voisinage de $\underline{q}^{(k-1)}$ à partir de la loi a posteriori $p(\underline{q}|\underline{Y},\varepsilon^{(k)},P^{(k-1)},M^{(k)},\underline{Cq}^{(k-1)},Cp^{(k-1)})$,
- générer un échantillon $P^{(k)}$ à partir de la loi a posteriori $p(P|\underline{Y},\varepsilon^{(k)},\underline{q}^{(k)},M^{(k-1)},\underline{Cq}^{(k-1)},C_p^{(k-1)})$,
- définir le voisinage du paramètre M à partir de la valeur $M^{(k-1)}$,
- générer un échantillon $M^{(k)}$ dans le voisinage de $M^{(k-1)}$ à partir de la loi a posteriori $p(M|\underline{Y},\varepsilon^{(k)},q^{(k)},P^{(k)},\underline{Cq}^{(k-1)},Cp^{(k-1)})$,
- définir le voisinage du paramètre $\underline{Cq}$ à partir de la valeur $\underline{Cq}^{(k-1)}$,
- générer un échantillon $\underline{Cq}^{(k)}$ dans le voisinage de $\underline{Cq}^{(k-1)}$ à partir de la loi a posteriori $p(\underline{Cq}|\underline{Y},\varepsilon^{(k)},\underline{q}^{(k)},P^{(k)},M^{(k)},Cp^{(k-1)})$, et
- générer un échantillon $Cp^{(k)}$ à partir de la loi a posteriori $p(Cp|\underline{Y},\varepsilon^{(k)},\underline{q}^{(k)},P^{(k)},M^{(k)},\underline{Cq}^{(k)})$.

En outre, à chaque itération, la loi jointe marginalisée est estimée et sa valeur est conservée en mémoire avec les valeurs courantes $\varepsilon^{(k)}$, $\underline{q}^{(k)}$, $P^{(k)}$, $M^{(k)}$, $\underline{Cq}^{(k)}$ et $Cp^{(k)}$.

Au bout d'un nombre prédéterminé K d'itérations, $1 \le K \le kmax$, un test 406 est exécuté sur la valeur courante de la loi jointe marginalisée. Si cette valeur courante reste inférieure à la dernière valeur stockée en mémoire à l'étape 402, alors l'étape 404 est interrompue et on passe à une étape 408 lors de laquelle les dernières valeurs de paramètres stockées en mémoire à l'étape 402 sont restaurées avec une probabilité dépendant des valeurs de la probabilité de la loi jointe marginalisée avant et après l'échantillonnage 402 du paramètre C. L'étape 408 met fin à la boucle d'échantillonnage 400 et est suivie de l'étape 108. Si la valeur courante de la loi jointe marginalisée testée à l'étape 406 est supérieure à la dernière valeur stockée en mémoire à l'étape 402, alors l'étape 404 est menée à son terme et suivie d'un retour à l'étape 402.

L'étape d'estimation 108 fournit des valeurs estimées $\hat{\varepsilon}, \hat{\underline{q}}, \hat{P}, \hat{M}, \hat{\underline{Cq}}, \hat{Cp}$ et $\hat{C}$ pour chacun des paramètres inconnus $\varepsilon$, $\underline{q}$, P, M, $\underline{Cq}$, Cp et C. On en déduit directement une valeur estimée $\underline{m} = \hat{M}(\hat{\underline{Cq}}) \cdot \hat{\underline{q}}$ pour le paramètre $\underline{m}$.

La phase suivante de reconstruction de spectre 200 est inchangée.

En variante du troisième mode de réalisation qui vient d'être décrit, un calibrage interne peut être réalisé en incorporant dans l'échantillon E, à l'étape 102, un échantillon E* de composants connus alourdis de marquage.

Une première conséquence d'un tel calibrage interne réalisé pendant l'étape de mesure 102 est d'ajouter des connaissances a priori sur au moins une partie des paramètres de la chaîne de traitement 12. En particulier, la loi a priori sur le nombre C de classes devient nécessairement nulle en dessous d'un minimum connu en fonction de l'échantillon de composants alourdis E* : ce minimum correspond au nombre de classes de composants alourdis. En outre, cela induit également une connaissance sur le vecteur M qui doit contenir les indications correspondantes sur les masses des composants alourdis de l'échantillon E*. Il est alors nécessaire de séparer le vecteur M en deux sous vecteurs : un vecteur $M_i$ des composants de l'échantillon E, remis en cause à chaque échantillonnage, et un vecteur $M_c$ des composants alourdis de l'échantillon E*, jamais remis en cause.

Une autre conséquence concerne l'étape 206, l'inverse du gain utilisé pour pondérer l'histogramme du paramètre $\underline{m}$ n'étant pas connu a priori mais estimé par le dispositif de traitement 14 grâce au calibrage interne.

Il apparaît clairement qu'un procédé tel que celui décrit précédemment, mis en oeuvre par le dispositif d'estimation 10, permet, grâce à une modélisation probabiliste fine de la chaîne de traitement 12, de fournir une estimation fiable d'un paramètre de masse moléculaire de composants d'un échantillon à analyser. En particulier, ce procédé excelle à évaluer individuellement les masses des composants de l'échantillon, ce que les méthodes d'analyse classiques réalisent moins bien.

Des applications concrètes de ce procédé comportent notamment la détection de marqueurs cancéreux (dans ce cas les composants sont des protéines) dans un échantillon biologique de sang ou d'urine.

On notera par ailleurs que l'invention n'est pas limitée aux modes de réalisation décrits précédemment. Il apparaîtra en effet à l'homme de l'art que diverses modifications peuvent être apportées au mode de réalisation décrit ci-dessus, à la lumière de l'enseignement qui vient de lui être divulgué.

Notamment, les composants d'intérêt ne sont pas nécessairement des protéines, mais peuvent être plus généralement des molécules ou des assemblages moléculaires pour une analyse biologique ou chimique.

En variante également, d'autres modèles probabilistes avec d'autres paramètres peuvent être choisis que ceux présentés en relation avec les figures 2, 4 et 5. En particulier, dans le troisième mode de réalisation, en changeant la définition du paramètre vectoriel $\underline{q}$ pour qu'il intègre lui-même une information sur les classes, il devient éventuellement possible de se passer des paramètres $Cq$ et $Cp$.

En variante également, il est possible de remettre en question l'hypothèse des gains Ge, Gf, Gc indépendants de la masse des molécules d'une manière relativement simple : si l'on dispose de plusieurs références de concentration, que ce soit lors d'un calibrage externe ou interne, on peut estimer chacun des gains supposés pour chacune des références, puis interpoler les valeurs de gains obtenues (par des splines, par exemple). La courbe résultante peut être inversée pour servir à multiplier l'histogramme de $\underline{m}$ lors de l'étape 206.

En variante également, lors de l'exécution d'une boucle d'échantillonnage de Gibbs, que ce soit au cours de l'étape 106 ou de l'étape 404, il est possible de remettre en cause le résultat de chaque itération. Par exemple, lors de l'estimation de la loi jointe marginalisée en fin d'itération, en comparant cette nouvelle estimation à la valeur précédente, si la valeur est plus faible, on accepte le nouveau jeu de paramètres avec une probabilité égale au rapport de la nouvelle probabilité sur l'ancienne probabilité. On peut aussi envisager une variante selon laquelle les paramètres sont systématiquement acceptés au cours d'une itération d'échantillonnage et où l'ensemble des paramètres est remis en cause à la fin de l'itération.

En variante également, dans les premier et troisième modes de réalisation, dans lesquels les paramètres $\underline{\mu}$ et M sont des vecteurs, l'échantillonnage de Gibbs peut, au lieu de se faire globalement sur l'ensemble du vecteur, se faire composante par composante, soit de la première à la dernière, soit dans autre ordre prédéterminé, soit en parcourant les composantes vectorielles dans un ordre aléatoire pour qu'un sens de parcours prédéterminé n'influe pas sur les résultats de l'échantillonnage.

En variante également, un processus de fusion d'adsorptions peut être engagé pour certaines composantes du paramètre $\underline{q}$, par exemple par élection stochastique des composantes concernées. Lors d'un tel processus de fusion, on fusionne chaque couple d'adsorptions suffisamment proches. De plus, il est possible :

- dans le premier mode de réalisation, de remplacer la masse associée (dans le vecteur $\underline{\mu}$) à la nouvelle adsorption créée par la somme des deux anciennes masses estimées pour les deux adsorptions fusionnées,
- dans les deuxième et troisième modes de réalisation, d'augmenter respectivement $M_0$ et M d'une certaine quantité, par exemple du rapport entre le nombre d'adsorptions et le nombre de composantes auxquelles on retranche 1 dans le paramètre $\underline{q}$.

Plus généralement, dans les revendications qui suivent, les termes utilisés ne doivent pas être interprétés comme limitant les revendications aux modes de réalisation exposés dans la présente description, mais doivent être interprétés pour y inclure tous les équivalents que les revendications visent à couvrir du fait de leur formulation et dont la prévision est à la portée de l'homme de l'art en appliquant ses connaissances générales à la mise en oeuvre de l'enseignement qui vient de lui être divulgué.

**Revendications**

1. Procédé d'estimation d'un paramètre de masse moléculaire ($\underline{m}$, Sp) dans un échantillon (E) comportant au moins un composant d'une masse moléculaire donnée, comportant les étapes suivantes :

- faire passer (102) l'échantillon (E) par une chaîne de traitement (12) comportant un spectromètre de masse (22) à capteur électromécanique de type MEMS ou NEMS, pour compter le nombre de détections successives de l'adsorption dudit composant,
- obtenir ainsi un signal ($\underline{Y}$) représentatif du paramètre de masse moléculaire ($\underline{m}$, Sp), et
- estimer (104, 106, 108, 200) le paramètre de masse moléculaire ($\underline{m}$, Sp) à l'aide d'un dispositif (14) de traitement de signal,

**caractérisé en ce que** le paramètre de masse moléculaire ($\underline{m}$, Sp) est défini sur la base d'un paramètre ($\underline{q}$, $\underline{m}$) de répartition temporelle de détections successives, par le capteur électromécanique de type MEMS ou NEMS, de l'adsorption dudit composant, et **en ce que** son estimation (104, 106, 108, 200) est réalisée par inférence bayésienne, sur la base d'une modélisation analytique directe dudit signal ($\underline{Y}$) en fonction du paramètre de masse moléculaire et de paramètres techniques (Ge, Gf, Gc, $\underline{f}$, c, $\underline{g}$, $\underline{h}$, $\varepsilon$) de la chaîne de traitement (12) comportant au moins un paramètre technique (c) du capteur électromécanique de type MEMS ou NEMS.

2. Procédé d'estimation d'au moins un paramètre de masse moléculaire selon la revendication 1, dans lequel la modélisation analytique comprend un paramètre ($\underline{q}$) représentant les instants, dits instants de détection, auxquels le capteur électromécanique détecte l'adsorption dudit composant, le paramètre de masse moléculaire ($\underline{m}$, Sp) étant défini à partir de ce paramètre représentant les instants de détection.

3. Procédé d'estimation d'au moins un paramètre de masse moléculaire selon la revendication 2, dans lequel le paramètre ($\underline{q}$) représentant les instants de détection prend la forme d'un vecteur ou d'une liste des paramètres de chaque détection de composant.

4. Procédé d'estimation d'au moins un paramètre de masse moléculaire selon l'une quelconque des revendications 1 à 3, comportant en outre une étape de détection des lieux d'adsorption des composants sur le capteur électromécanique de type MEMS ou NEMS, dans lequel la modélisation analytique comporte en outre un paramètre de répartition temporelle de ces lieux d'adsorption et une fonction déterministe donnant une valeur de chute de fréquence pour chaque couple de valeurs d'une masse de composant adsorbée et d'un lieu d'adsorption correspondant.

5. Procédé d'estimation d'au moins un paramètre de masse moléculaire selon l'une quelconque des revendications 1 à 4, dans lequel au moins deux des paramètres de masse moléculaire ($\underline{m}$, Sp) et de la chaîne de traitement (Ge, Gf, Gc, $\underline{f}$, c, $\underline{g}$, $\underline{h}$, $\varepsilon$) en fonction desquels la modélisation analytique directe dudit signal ($\underline{Y}$) est définie ont une relation de dépendance probabiliste entre eux, et dans lequel le traitement de signal par inférence bayésienne est en outre réalisé sur la base d'une modélisation par loi de probabilité a priori conditionnelle de cette dépendance.

6. Procédé d'estimation d'au moins un paramètre de masse moléculaire selon l'une quelconque des revendications 1 à 5, dans lequel l'étape (104, 106, 108, 200) d'estimation du paramètre de masse moléculaire ($\underline{m}$, Sp) comporte, par approximation de la loi de probabilité a posteriori jointe d'un paramètre ($\mu$, $M_0$, M) lié au paramètre de masse moléculaire ($\underline{m}$, Sp) et des paramètres techniques de la chaîne de traitement (12) conditionnellement au signal obtenu ($\underline{Y}$) à l'aide d'un algorithme d'échantillonnage stochastique :

- une boucle d'échantillonnage (106 ; 400) de ces paramètres, fournissant des valeurs échantillonnées de ces paramètres, et
- une estimation (108, 200) du paramètre de masse moléculaire ($\underline{m}$, Sp) calculée à partir des valeurs échantillonnées fournies.

7. Procédé d'estimation d'au moins un paramètre de masse moléculaire selon les revendications 3 et 6, dans lequel :

- le paramètre ($\underline{q}$) représentant les instants de détection prend la forme d'un vecteur à composantes binaires, l'une des valeurs binaires, A, indiquant la détection d'une adsorption,
- à chaque itération de la boucle d'échantillonnage (106 ; 400), un échantillon de ce paramètre est calculé dans un voisinage de l'échantillon calculé à l'itération précédente,
- le voisinage d'un échantillon courant de ce paramètre est défini de la façon suivante : tout échantillon comportant une composante à A en plus ou en moins, une composante à A décalée, ou une composante A regroupant deux composantes à A successives de l'échantillon courant.

8. Procédé d'estimation d'au moins un paramètre de masse moléculaire selon la revendication 6 ou 7, dans lequel :

- à chaque itération de la boucle d'échantillonnage (106 ; 400), une probabilité jointe d'au moins tous les paramètres échantillonnés est estimée, et
- l'estimation du paramètre de masse moléculaire ($\underline{m}$, Sp) se fait, conjointement avec celle des paramètres échantillonnés, sur la base des valeurs successives de ladite probabilité jointe.

9. Procédé d'estimation d'au moins un paramètre de masse moléculaire selon l'une quelconque des revendications 6 à 8, dans lequel le paramètre de masse moléculaire est un spectre de masse (Sp) relatif à au moins un composant dont la masse fait partie des paramètres traités par la boucle d'échantillonnage (106 ; 400) et est l'un des éléments de l'ensemble constitué d'une unique masse moléculaire ($M_0$), d'une pluralité discrète de masses moléculaires (M) et d'une distribution continue de masses ($\underline{\mu}$).

10. Procédé d'estimation d'au moins un paramètre de masse moléculaire selon l'une quelconque des revendications 1 à 9, dans lequel le paramètre de masse moléculaire ($\underline{m}$, Sp) est relatif à des protéines et l'échantillon (E) comporte l'un des éléments de l'ensemble constitué de sang, plasma, urine, fluide biologique et lysat d'un échantillon biologique.

11. Dispositif (10) d'estimation d'un paramètre de masse moléculaire ($\underline{m}$, Sp) dans un échantillon (E) comportant au moins un composant d'une masse moléculaire donnée, comportant :

- une chaîne (12) de traitement de l'échantillon (E) comportant un spectromètre de masse (22) à capteur électromécanique de type MEMS ou NEMS, conçue pour compter le nombre de détections successives de l'adsorption dudit composant et fournir un signal ($\underline{Y}$) représentatif du paramètre de masse moléculaire,
- un dispositif (14) de traitement de signal conçu pour appliquer, en combinaison avec la chaîne de traitement (12), un procédé d'estimation de paramètre de masse moléculaire selon l'une quelconque des revendications 1 à 10, le paramètre de masse moléculaire ($\underline{m}$, Sp) étant défini sur la base d'un paramètre ($\underline{q}$, m) de répartition temporelle de détections successives, par le capteur électromécanique de type MEMS ou NEMS, de masses de composants adsorbés.

**Patentansprüche**

1. Verfahren zur Schätzung eines Molekularmasseparameters ($\underline{m}$, Sp) in einer Probe (E) mit mindestens einem Bestandteil einer gegebenen Molekularmasse, das die folgenden Schritte umfasst:

- die Probe (E) eine Verarbeitungskette (12) mit Massenspektrometer (22) mit elektromechanischem Sensor des Typs MEMS oder NEMS durchlaufen lassen (102), um die Anzahl der aufeinanderfolgenden Detektionen der Adsorption des genannten Bestandteils zählen,
- damit ein Signal ($\underline{Y}$) zu gewinnen, das für den Molekularmasseparameter ($\underline{m}$, Sp) repräsentativ ist, und
- den Molekularmasseparameter ($\underline{m}$, Sp) mithilfe einer Vorrichtung (14) zur Zeichenverarbeitung zu schätzen (104, 106, 108, 200),

**gekennzeichnet dadurch, dass** der Molekularmasseparameter ($\underline{m}$, Sp) auf der Grundlage eines Parameters ($\underline{q}$, $\underline{m}$) für die zeitliche Verteilung der aufeinanderfolgenden Detektionen der Adsorption des genannten Bestandteils durch den elektromechanischen Sensor des Typs MEMS oder NEMS definiert wird, und dass dessen Schätzung (104, 106, 108, 200) durch bayessche Inferenz auf der Grundlage einer direkten analytischen Modellierung des genannten Signals ($\underline{Y}$) in Abhängigkeit vom Molekularmasseparameter und von technischen Parametern (Ge, Gf, Gc, $\underline{f}$, c, $\underline{g}$, $\underline{h}$, $\varepsilon$) der Verarbeitungskette (12) mit mindestens einem technischen Parameter (c) des elektromechanischen Sensors des Typs MEMS oder NEMS erfolgt.

2. Verfahren zur Schätzung von mindestens einem Molekularmasseparameter gemäß Anspruch 1, in dem die analytische Modellierung einen Parameter ($\underline{q}$) umfasst, der die Momente, die sogenannten Detektionsmomente, repräsentiert, in denen der elektromechanische Sensor die Adsorption des genannten Bestandteils erkennt, wobei der Molekularmasseparameter ($\underline{m}$, Sp) ausgehend von diesem die Detektionsmomente repräsentierenden Parameter definiert wird.

3. Verfahren zur Schätzung von mindestens einem Molekularmasseparameter gemäß Anspruch 2, in dem der die Detektionsmomente repräsentierende Parameter ($q$) die Form eines Vektors oder einer Liste der Parameter jeder Bestandteildetektion aufweist.

4. Verfahren zur Schätzung von mindestens einem Molekularmasseparameter gemäß einem der Ansprüche 1 bis 3, das ausserdem ein Schritt der Detektion der Adsorptionsstellen der Bestandteile auf dem elektromechanischen Sensor des Typs MEMS oder NEMS umfasst, in der die analytische Modellierung ausserdem einen Parameter für die zeitliche Verteilung dieser Adsorptionsstellen und eine determinierende Funktion umfasst, die einen Frequenzabfallwert für jedes Wertepaar einer adsorbierten Bestandteilmasse und einer entsprechenden Adsorptionsstelle umfasst.

5. Verfahren zur Schätzung von mindestens einem Molekularmasseparameter gemäß einem der Ansprüche 1 bis 4, in dem mindestens zwei der Parameter der Molekularmasse ($m$, Sp) und der Verarbeitungskette (Ge, Gf, Gc, $f$, c, $g$, $h$, $\varepsilon$), abhängig von denen die direkte analytische Modellierung des genannten Signals ($Y$) definiert wird, eine probabilistische Abhängigkeit zueinander aufweisen, und in dem die Signalverarbeitung durch bayessche Inferenz ausserdem auf der Grundlage einer Modellbildung durch Wahrscheinlichkeitsgesetz erfolgt, das a priori eine Bedingung dieser Abhängigkeit ist.

6. Verfahren zur Schätzung von mindestens einem Molekularmasseparameter gemäß einem der Ansprüche 1 bis 5, in dem der Schritt (104, 106, 108, 200) zur Schätzung des Molekularmasseparameters ($m$, Sp) durch Näherung des Wahrscheinlichkeitsgesetzes, das a posteriori einem Parameter ($\mu$, $M_0$, M) hinzugefügt wird, der mit dem Molekularmasseparameter ($m$, Sp) und den technischen Parametern der Verarbeitungskette (12) verbunden ist, bedingt durch das mithilfe eines stochastischen Probenahmealgorithmus erzielte Signal ($Y$) umfasst:

- Probenahmeschleife (106; 400) dieser Parameter, die Probenahmewerte dieser Parameter bereitstellt, und
- Schätzung (108, 200) des Molekularmasseparameters ($m$, Sp), die auf der Grundlage der bereitgestellten Probenahmewerte berechnet wird.

7. Verfahren zur Schätzung mindestens eines Molekularmasseparameters gemäß den Ansprüchen 3 und 6, in dem:

- der die Detektionsmomente repräsentierende Parameter ($g$) die Form eines Vektors mit binären Werten aufweist, wobei einer der Binärwerte, A, die Detektion einer Adsorption angibt,
- an jeder Iteration der Probenahmeschleife (106; 400) eine Probe dieses Parameters in unmittelbarer Nähe der bei der vorherigen Iteration berechneten Probe berechnet wird,
- die unmittelbare Nähe einer aktuellen Probe dieses Parameters folgendermaßen definiert ist: Jede Probe mit einem Bestandteil für A mehr oder weniger, einem versetzten Bestandteil für A, oder einem A-Bestandteil, der zwei aufeinanderfolgende Bestandteile für A der aktuellen Probe enthält.

8. Verfahren zur Schätzung mindestens eines Molekularmasseparameters gemäß den Ansprüchen 6 oder 7, in dem:

- an jeder Iteration der Probenahmeschleife (106; 400) eine verbundete Wahrscheinlichkeit mindestens aller beprobten Parameter geschätzt wird, und
- die Schätzung des Molekularmasseparameters ($m$, Sp) gemeinsam mit der Schätzung der beprobten Parameter auf der Grundlage der aufeinanderfolgenden Werte der genannten hinzugefügten Wahrscheinlichkeit erfolgt.

9. Verfahren zur Schätzung mindestens eines Molekularmasseparameters gemäß einem der Ansprüche 6 bis 8, in dem der Molekularmasseparameter ein Massespektrum (Sp) in Bezug auf zumindest einen Bestandteil ist, dessen Masse zu den durch die Probenahmeschleife verarbeiteten Parametern gehört (106; 400) und eines der Elemente der aus einer einzigen Molekularmasse ($M_0$), einer diskreten Vielzahl von Molekularmassen (M) und einer kontinuierlichen Masseverteilung ($\mu$) gebildeten Gesamtheit ist.

10. Verfahren zur Schätzung mindestens eines Molekularmasseparameters gemäß einem der Ansprüche 1 bis 9, in dem der Molekularmasseparameter ($m$, Sp) auf Proteine bezogen ist und die Probe (E) ein Element der aus Blut, Plasma, Urin, biologischer Flüssigkeit und Lysat einer biologischen Probe gebildeten Gesamtheit enthält.

**11.** Vorrichtung zur Schätzung eines Molekularmasseparameters (m, Sp) in einer Probe (E) mit mindestens einem Bestandteil einer gegebenen Molekularmasse, die umfasst:

- einer Kette (12) zur Verarbeitung der Probe (E) mit Massenspektrometer (22) mit elektromechanischer Sonde des Typs MEMS oder NEMS, die darauf ausgelegt ist, die Anzahl der aufeinanderfolgenden Absorptionsdetektionen für den genannten Bestandteil zu zählen und ein das den Molekularmasseparameter repräsentierendes Signal (Y) bereitzustellen,
- einer Vorrichtung (14) zur Signalverarbeitung, die darauf ausgelegt ist, in Kombination mit der Verarbeitungskette (12) ein Verfahren zur Schätzung von Molekularmasseparametern gemäß einem der Ansprüche 1 bis 10 anzuwenden, wobei der Molekularmasseparameter (m, Sp) auf der Grundlage eines Parameters (q, m) für die zeitliche Verteilung der aufeinanderfolgenden adsorbierten Bestandteilmassen durch den elektromechanischen Sensor des Typs MEMS oder NEMS definiert wird.

**Claims**

**1.** Method for estimating a molecular mass parameter (m, Sp) in a sample (E) that includes at least one component with a given molecular mass, comprising the following steps:

- passing (102) the sample (E) through a processing chain (12) comprising a mass spectrometer (22) with a MEMS or NEMS electromechanical sensor, in order to count the number of successive detections of said component's adsorption,
- in this way obtaining a signal (Y) representing the molecular mass parameter (m, Sp), and
- estimating (104, 106, 108, 200) the molecular mass parameter (m, Sp) by means of a signal processing device (14),

**characterized in that** the molecular mass parameter (m, Sp) is defined on the basis of a parameter (q, m) of time distribution of successive detections, by the MEMS or NEMS electromechanical sensor, of the adsorption of said component, and **in that** the estimation (104, 106, 108, 200) thereof is made by Bayesian inference, on the basis of a direct analytical modeling of said signal (Y) according to the molecular mass parameter and to technical parameters (Ge, Gf, Gc, f, c, g, h, $\varepsilon$) of the processing chain (12) comprising at least one technical parameter (c) of the MEMS or NEMS electromechanical sensor.

**2.** Method for estimating at least one molecular mass parameter according to claim 1, wherein the analytical modeling comprises a parameter (q) representing the instants, referred to as the detection instants, at which the electromechanical sensor detects the adsorption of said component, the molecular mass parameter (m, Sp) being defined on the basis of said parameter representing the detection instants.

**3.** Method for estimating at least one molecular mass parameter according to claim 2, wherein the parameter (q) representing the detection instants takes the form of a vector or of a list of parameters of each component detection.

**4.** Method for estimating at least one molecular mass parameter according to any one of claims 1 to 3, further comprising a step for detecting the adsorption sites of components on the MEMS or NEMS electromechanical sensor, wherein the analytical modeling further comprises a parameter of time distribution of said adsorption sites and a deterministic function that returns a value of drop in frequency for each pair of values for a mass of adsorbed component and for a corresponding adsorption site

**5.** Method for estimating at least one molecular mass parameter according to any one of claims 1 to 4, wherein at least two of the molecular mass parameters (m, Sp) and the processing chain parameters (Ge, Gf, Gc, f, c, g, h, $\varepsilon$) according to which the direct analytical modeling of said signal (Y) is defined have a probabilistic dependency relationship with each other, and wherein the signal processing by Bayesian inference is further carried out on the basis of a modeling by a conditional prior probability law of this dependency.

**6.** Method for estimating at least one molecular mass parameter according to any one of claims 1 to 5, wherein the step (104, 106, 108, 200) of estimating the molecular mass parameter (m, Sp) comprises, by approximation of the joint posterior probability law of a parameter ($\mu$, $M_0$, M) linked to the molecular mass parameter (m, Sp) and the technical parameters of the processing chain (12) conditionally to the signal obtained (Y) by means of a stochastic sampling algorithm:

- a loop (106; 400) for sampling these parameters, supplying sampled values of these parameters, and
- an estimation (108, 200) of the molecular mass parameter ($\underline{m}$, Sp) calculated from the sampled values supplied.

7. Method for estimating at least one molecular mass parameter according to claims 3 and 6, wherein:

- the parameter ($\underline{q}$) representing the detection instants takes the form of a vector with binary components, one of the binary values, A, indicating the detection of an adsorption,
- at each iteration of the sampling loop (106; 400), a sample of this parameter is calculated in a neighborhood of the sample calculated at the previous iteration,
- the neighborhood of a current sample of this parameter is defined in the following way: any sample comprising a component with A plus or minus, a component with A shifted, or a component A grouping together two successive components with A of the current sample.

8. Method for estimating at least one molecular mass parameter according to claim 6 or 7, wherein:

- at each iteration of the sampling loop (106; 400), a joint probability of at least all the sampled parameters is estimated, and
- the estimation of the molecular mass parameter ($\underline{m}$, Sp) is done, conjointly with that of the sampled parameters, on the basis of the successive values of said joint probability.

9. Method for estimating at least one molecular mass parameter according to any one of claims 6 to 8, wherein the molecular mass parameter is a mass spectrum (Sp) relating to at least one component the mass of which forms part of the parameters processed by the sampling loop (106; 400) and is one of the elements of the set consisting of a single molecular mass ($M_0$), a discrete plurality of molecular masses (M) and a continuous distribution of masses ($\underline{\mu}$).

10. Method for estimating at least one molecular mass parameter according to any one of claims 1 to 9, wherein the molecular mass parameter ($\underline{m}$, Sp) relates to proteins and the sample (E) comprises one of the elements of the set consisting of blood, plasma, urine, biological fluid and lysate of a biological sample.

11. Device (10) for estimating a molecular mass parameter ($\underline{m}$, Sp) in a sample (E) that includes at least one component with a given molecular mass, comprising:

- a chain (12) for processing the sample (E) comprising a mass spectrometer (22) with a MEMS or NEMS electromechanical sensor, designed for counting the number of successive detections of said component's adsorption and supplying a signal ($\underline{Y}$) representing the molecular mass parameter,
- a signal processing device (14) designed to apply, in combination with the processing chain (12), a molecular mass parameter estimation method according to any one of claims 1 to 10, wherein the molecular mass parameter ($\underline{m}$, Sp) is defined on the basis of a parameter ($\underline{q}$, $\underline{m}$) of time distribution of successive detections, by the MEMS or NEMS electromechanical sensor, of adsorbed components' masses.

Figure 1

Figure 2

# *Figure 3*

## Figure 4

## Figure 5

## Figure 6

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2007023621 A

**Littérature non-brevet citée dans la description**

- **NAIK et al.** Towards single-molecule nanomechanical mass spectrometry. *Nature Nanotechnology,* Juillet 2009, vol. 4, 445-450
- **GRUBER D et al.** Counting of obscure events: A Bayesian approach. *CHEMICAL PHYSICS LETTERS,* 04 Juin 2009, vol. 474 (4-6), ISSN 0009-2614, 371-374

- **DOHN et al.** Mass and position determination of attached particles on cantilever based mass sensors. *Review of scientific intruments,* 31 Octobre 2007, vol. 78, 103303